(19) Europäisches Patentamt
European Patent Office
Office européen des brevets

(11) EP 2 511 276 A1

(12) EUROPEAN PATENT APPLICATION
published in accordance with Art. 153(4) EPC

(43) Date of publication:
**17.10.2012 Bulletin 2012/42**

(21) Application number: **10804402.5**

(22) Date of filing: **27.07.2010**

(51) Int Cl.:
***C07D 487/04*** (2006.01) ***A61K 31/519*** (2006.01)
***A61P 35/00*** (2006.01) ***A61P 35/02*** (2006.01)
***A61P 43/00*** (2006.01)

(86) International application number:
**PCT/JP2010/062602**

(87) International publication number:
**WO 2011/013656 (03.02.2011 Gazette 2011/05)**

(84) Designated Contracting States:
**AL AT BE BG CH CY CZ DE DK EE ES FI FR GB GR HR HU IE IS IT LI LT LU LV MC MK MT NL NO PL PT RO SE SI SK SM TR**

(30) Priority: **28.07.2009 JP 2009175619**

(71) Applicant: **Ube Industries, Ltd.**
**Ube City,**
**Yamaguchi (JP)**

(72) Inventors:
- **TSUBOIKE, Kazunari**
  **Yamaguchi 755-8633 (JP)**
- **MIZUNO, Gen**
  **Yamaguchi 755-8633 (JP)**
- **NISHIDA, Hiroshi**
  **Yamaguchi 755-8633 (JP)**
- **KONO, Shigeyuki**
  **Yamaguchi 755-8633 (JP)**
- **TOYOTA, Akiko**
  **Tokyo 134-0081 (JP)**
- **TANZAWA, Fumie**
  **Tokyo 134-0081 (JP)**
- **FUJIWARA, Kosaku**
  **Tokyo 134-0081 (JP)**

(74) Representative: **Kuhnen & Wacker**
**Patent- und Rechtsanwaltsbüro**
**Prinz-Ludwig-Strasse 40A**
**85354 Freising (DE)**

# (54) PYRROLO[2,3-D]PYRIMIDINE DERIVATIVE

(57) Provided is a compound represented by the Formula (I) having a HER2 inhibitory action or a pharmacologically acceptable salt thereof,

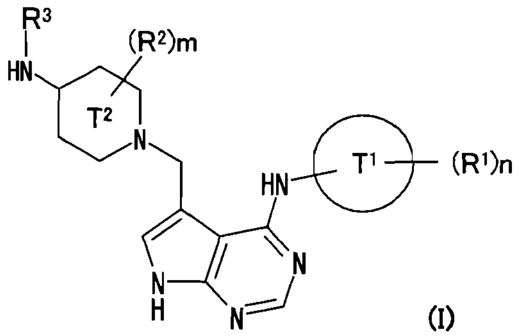

(I)

wherein $T^1$ is a phenyl group, an indazolyl group, or a benzofuryl group, n is an integer of 0 to 3, $R^1$ is a hydrogen atom, a halogen atom, a $C_{1-4}$ alkyl group, a $C_{2-6}$ alkenyl group, a $C_{2-6}$ alkynyl group, or a $C_{1-4}$ alkoxy group, m is an integer of 0 to 3, $R^2$ is a hydroxy group or a $C_{1-4}$ alkyl group optionally substituted with one or two $C_{1-4}$ alkoxy groups, and $R^3$ is a hydrogen atom, a $C_{1-4}$ alkyl group, or a $C_{2-6}$ alkynyl group.

EP 2 511 276 A1

## Description

### TECHNICAL FIELD

The present invention relates to a novel compound having an inhibitory action on HER2 kinase activity, a pharmacologically acceptable salt thereof, or a pharmaceutical composition comprising them as an active ingredient.

### BACKGROUND ART

A HER2 gene, i.e., a Human EGF Related Receptor-2 gene is a gene of a transmembrane receptor protein having a homology with the epidermal growth factor (EGF) receptor (EGFR), and these receptors have tyrosine kinase activities. It has been observed that the HER2 protein (hereinafter referred to as HER2) is overexpressed in human breast cancer cells and its expression level correlates to a prognosis (Non-Patent Document 1). Furthermore, HER2 is also observed to be overexpressed in various cancers such as gastric cancer, non-small cell lung cancer, pancreatic cancer, ovarian cancer and the like (Non-Patent Document 2). In the downstream of HER2, there exists intracellular signal transduction pathways such as Akt and MAPK which are strongly associated with cell growth, and it is considered that activation of these signals by the overexpression of HER2 results in a promotion of cancer growth. In fact, it has been observed that the overexpression of HER2 in mouse fibroblast leads to an enhanced cell growth potential (Non-Patent Document 3).

Also, a human monoclonal antibody against HER2 called trastuzumab (trade name Herceptin) and a small molecule which directly inhibits the tyrosine kinase activity of HER2 called lapatinib (trade name Tykerb) have been demonstrated in clinical trials to be effective to an advanced breast cancer patient with HER2 overexpression. Therefore, both agents are used as therapeutic agents for the advanced breast cancer patient with HER2 overexpression, and the validity of HER2 as a target of anticancer agents has already been demonstrated in clinical scenes. However, it has also been found that said HER2 signal inhibitors exhibit no drug efficacy on many breast cancer patients with HER2 overexpression, and the development of an agent having more potent HER2 kinase inhibitory activity (hereinafter referred also to as "HER2 inhibitory activity") is urgently needed (Non-Patent Document 4).

Pyrrolo[2,3-d]pyrimidine derivatives are disclosed as compounds having HER2 kinase inhibitory activity (hereinafter referred also to as "HER2 inhibitors") in Patent Document 1 and Patent Document 2, but a compound of the present invention is not disclosed.

### PRIOR ART DOCUMENTS

### PATENT DOCUMENTS

Patent Document 1: WO 1997/02266 pamphlet (US patent No. 6140332)
Patent Document 2: WO 1998/07726 pamphlet (US patent No. 6180636)

### NON-PATENT DOCUMENTS

Non-Patent Document 1: Stem Cells, vol. 16, No. 6, 413-428, 1998
Non-Patent Document 2: Annals Of Oncology, vol. 12, No. S1, S81-587, 2001
Non-Patent Document 3: Proc Natl Acad Sci U.S.A., vol. 84, No. 20, 7159-7163, 1987
Non-Patent Document 4: Cancer Treatment Reviews, vol. 35, No. 2, 121-136, 2009

### DISCLOSURE OF INVENTION

### PROBLEMS TO BE SOLVED BY THE INVENTION

The present inventors have studied extensively about a compound having a HER2 inhibitory activity to find that a compound of the Formula (I) of the present invention inhibits potently the HER2 kinase activity and exhibits an excellent cell growth inhibitory effect, and completed the present invention. In other words, the object of the present invention is to provide a compound having an excellent HER2 inhibitory activity, or a pharmacologically acceptable salt thereof. Another object of the present invention is to provide a pharmaceutical composition, or a HER2 inhibitor, especially an

anti-tumor agent comprising said compound, or a pharmacologically acceptable salt thereof as an active ingredient.

Although a compound of the present invention has an excellent HER2 inhibitory activity, said compound may also have a different kinase inhibitory activity, such as an inhibitory effect against angiogenesis-related protein kinase activity.

MEANS FOR SOLVING THE PROBLEMS

A compound of the present invention is characterized by having a piperidin-1-ylmethyl group at the 5-position of the pyrrolo[2,3-d]pyrimidine backbone, and having an optionally substituted amino group at the 4-position of said piperidyl group.

In other words, the present invention relates to,

(1) a compound of the Formula (I):

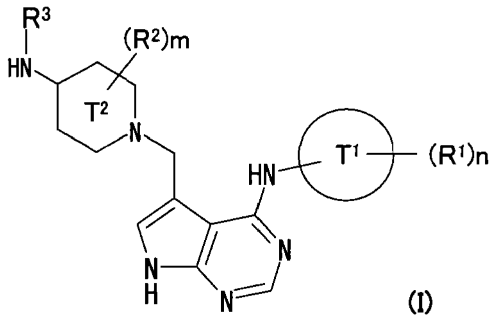

(I)

wherein,
$T^1$ is a phenyl group, an indazolyl group, or a benzofuryl group,
-$(R^1)$n represents that $T^1$ is substituted with the same or different n groups of $R^1$,
n is an integer of 0 to 3,
$R^1$ is a hydrogen atom, a halogen atom, a $C_{1-4}$ alkyl group, a $C_{2-6}$ alkenyl group, a $C_{2-6}$ alkynyl group, or a $C_{1-4}$ alkoxy group,
$T^2$ is a piperidinyl group,
-$(R^2)$m represents that $T^2$ is substituted with the same or different m groups of $R^2$,
m is an integer of 0 to 3,
$R^2$ is a hydrogen atom, a hydroxy group, or a $C_1$-$_4$ alkyl group, wherein said $C_{1-4}$ alkyl group may be substituted with one or two $C_{1-4}$ alkoxy groups,
$R^3$ is a hydrogen atom, a $C_{1-4}$ alkyl group, or a $C_{2-6}$ alkynyl group,
or a pharmacologically acceptable salt thereof,

(2) the compound according to the above (1), wherein $R^1$ is a hydrogen atom, a fluorine atom, a chlorine atom, a bromine atom, a methyl group, an ethyl group, a vinyl group, an allyl group, an ethynyl group, a 1-propynyl group, a 2-propynyl group, a methoxy group, or an ethoxy group,
(3) the compound according to the above (1) or (2), wherein $R^2$ is a hydrogen atom, a hydroxy group, a methyl group, an ethyl group, a methoxy group, an ethoxy group, a methoxymethyl group, an ethoxymethyl group, or a 2-methoxyethyl group,
(4) the compound according to any one of the above (1) to (3), wherein $R^3$ is a hydrogen atom, a methyl group, an ethyl group, or a 2-propynyl group,
(5) the compound according to any one of the above (1) to (3), wherein $R^3$ is a hydrogen atom,
(6) the compound according to the above (1), wherein the group of the following Formula (II) in the Formula (I),

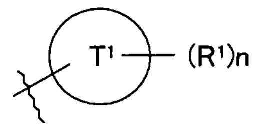

(II)

is the group of the following Formula (IIa),

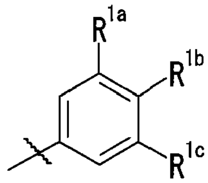

(IIa)

wherein $R^{1a}$, $R^{1b}$, and $R^{1c}$ are each the same or different and each is a hydrogen atom, a halogen atom, a $C_{1-4}$ alkyl group, a $C_{2-6}$ alkenyl group, a $C_{2-6}$ alkynyl group, or a $C_{1-4}$ alkoxy group, or a benzofuranyl group,

(7) the compound according to the above (6), wherein $R^{1a}$ is a halogen atom, a $C_{1-4}$ alkyl group, a $C_{2-6}$ alkenyl group, a $C_{2-6}$ alkynyl group, or a $C_{1-4}$ alkoxy group, and $R^{1b}$ and $R^{1c}$ are each a hydrogen atom,

(8) the compound according to the above (6), wherein $R^{1a}$ and $R^{1c}$ are each a hydrogen atom, and $R^{1b}$ is a halogen atom, a $C_{1-4}$ alkyl group, a $C_{2-6}$ alkenyl group, a $C_{2-6}$ alkynyl group, or a $C_{1-4}$ alkoxy group,

(9) the compound according to the above (6), wherein $R^{1a}$ and $R^{1b}$ are each the same or different and each is a halogen atom, a $C_{1-4}$ alkyl group, a $C_{2-6}$ alkenyl group, a $C_{2-6}$ alkynyl group, or a $C_{1-4}$ alkoxy group, and $R^{1c}$ is a hydrogen atom,

(10) the compound according to the above (1), wherein the group of the following Formula (III) in the Formula (I),

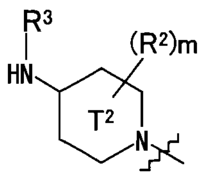

(III)

is the group of the following Formula (IIIa),

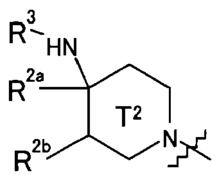

(IIIa)

wherein $R^{2a}$ and $R^{2b}$ are each the same or different and each is a hydrogen atom, a hydroxy group, or a $C_{1-4}$ alkyl group, wherein said $C_{14}$ alkyl group may be substituted with one or two $C_{1-4}$ alkoxy groups,

(11) the compound according to the above (10), wherein $R^{2a}$ and $R^{2b}$ are each the same or different and each is a hydrogen atom or a methyl group,

(12) the compound according to the above (10) or (11), wherein $R^3$ is a hydrogen atom,

(13) the compound according to the above (10), wherein the group of the Formula (III) is any one group selected from the following (IIIb) group,

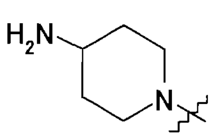
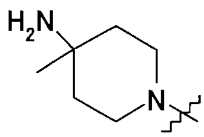
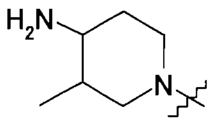
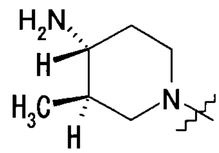
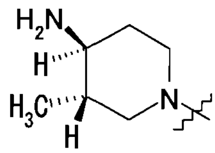

(IIIb)

,

(14) the compound according to the above (1), wherein the compound of the Formula (I) is any one compound of the following (IV) group,

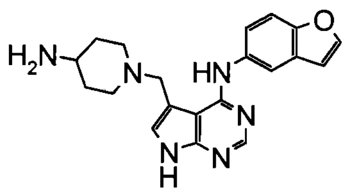
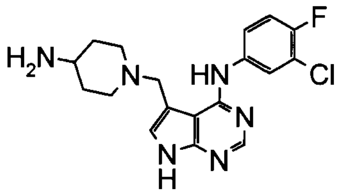
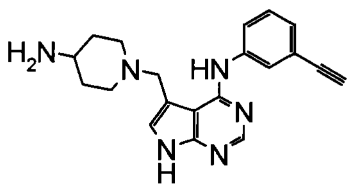
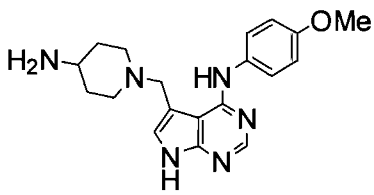
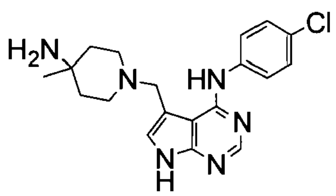
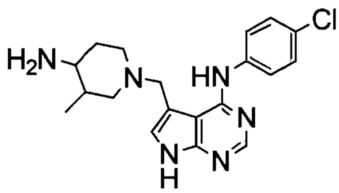
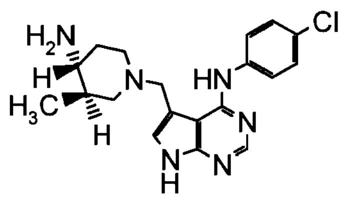
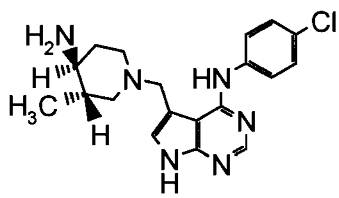

(IV)

,

(15) a pharmacologically acceptable salt of the compound according to any one of the above (2) to (14),

(16) a pharmaceutical composition comprising the compound according to any one of the above (1) to (14) or a pharmacologically acceptable salt thereof as an active ingredient,

(17) a HER2 inhibitor comprising the compound according to any one of the above (1) to (14) or a pharmacologically acceptable salt thereof as an active ingredient,

(18) an anti-tumor agent comprising the compound according to any one of the above (1) to (14) or a pharmacologically acceptable salt thereof as an active ingredient,

(19) the anti-tumor agent according to the above (18), wherein the tumor is a blood cancer such as leukemia,

lymphoma, or multiple myeloma, brain tumor, head and neck cancer, esophageal cancer, gastric cancer, appendix cancer, colon cancer, anal cancer, gallbladder cancer, bile duct cancer, pancreatic cancer, gastrointestinal stromal tumor, small cell lung cancer, non-small cell lung cancer, liver cancer, mesothelioma, thyroid cancer, kidney cancer, prostate cancer, neuroendocrine tumor, melanoma, breast cancer, endometrial cancer, cervical cancer, ovarian cancer, osteosarcoma, soft tissue sarcoma, Kaposi's sarcoma, myosarcoma, kidney cancer, bladder cancer, salivary gland cancer and/or testicular cancer.

Also, the present invention provides a method for prevention, treatment or prevention of recurrence of tumor consisting of administering the compound according to any one of the above (1) to (15) or pharmacologically acceptable salt thereof, the pharmaceutical composition according to the above (16), the HER2 inhibitor according to the above (17), or the anti-tumor agent according to the above (18) or (19) to a warm-blooded animal (preferably human).

EFFECT OF THE INVENTION

A compound of the Formula (I) of the present invention or a pharmacologically acceptable salt thereof has a potent HER2 inhibitory activity and exibits an excellent anti-tumor effect by inhibiting the intracellular phosphorylation of HER2 and suppressing the cell growth. Therefore, a compound of the present invention or a pharmacologically acceptable salt thereof, or a pharmaceutical composition comprising a compound of the present invention or a pharmacologically acceptable salt thereof as an active ingredient is useful as an anti-tumor agent, especially as a therapeutic agent for tumor wherein said tumor is, for example, a blood cancer such as leukemia, lymphoma, or multiple myeloma, brain tumor, head and neck cancer, esophageal cancer, gastric cancer, appendix cancer, colon cancer, anal cancer, gallbladder cancer, bile duct cancer, pancreatic cancer, gastrointestinal stromal tumor, small cell lung cancer, non-small cell lung cancer, liver cancer, mesothelioma, thyroid cancer, kidney cancer, prostate cancer, neuroendocrine tumor, melanoma, breast cancer, endometrial cancer, cervical cancer, ovarian cancer, osteosarcoma, soft tissue sarcoma, Kaposi's sarcoma, myosarcoma, kidney cancer, bladder cancer, salivary gland cancer and/or testicular cancer. Among the above tumors, a compound of the present invention or a pharmacologically acceptable salt thereof, or a pharmaceutical composition comprising a compound of the present invention or a pharmacologically acceptable salt thereof as an active ingredient is effective as a therapeutic agent for tumor with HER2 overexpression.

BEST MODE FOR CARRYING OUT THE INVENTION

As used herein, the term "$C_{1-4}$ alkyl group" means a straight or branched alkyl group having 1 to 4 carbon atoms, and can be exemplified such as a methyl group, an ethyl group, a propyl group, an isopropyl group, a butyl group, an isobutyl group, a sec-butyl group, a tert-butyl group and the like. The term "$C_{2-6}$ alkenyl group" means a monovalent group derived by the removal of one hydrogen atom from any one carbon atom of a straight or branched alkene having 2 to 6 carbon atoms, and can be exemplified such as a vinyl group, an allyl group, a 1-propenyl group, a 1-methylvinyl group, a 3-butenyl group, a 3-pentenyl group, a 4-hexenyl group and the like. The term "$C_{2-6}$ alkynyl group" means a monovalent group derived by the removal of one hydrogen atom from any one carbon atom of a straight or branched alkyne having 2 to 6 carbon atoms, and can be exemplified such as an ethynyl group, a 1-propynyl group, a 2-propynyl group, a 1-butynyl group, a 2-butynyl group, a 3-butynyl group, a 1-methyl-2-propynyl group, a 1-pentynyl group, a 2-pentynyl group, a 3-pentynyl group, a 4-pentynyl group, a 1-hexynyl group, a 2-hexynyl group, a 3-hexynyl group, a 4-hexynyl group, a 5-hexynyl group and the like. The term "halogen atom" can be exemplified such as a fluorine atom, a chlorine atom, a bromine atom, an iodine atom and the like.

As used herein, the term "$C_{1-4}$ alkoxy group" means a group consisting of said "$C_{1-4}$ alkyl group" and an oxygen atom, and can be exemplified such as a methoxy group, an ethoxy group, a propoxy group, an isopropoxy group and the like.

In the Formula (I), $T^1$ is a phenyl group, an indazolyl group, or a benzofuryl group. More preferably, $T^1$ is a phenyl group, a 1H-indazol-4-yl group, a 1H-indazol-5-yl group, a 1H-indazol-6-yl group, a 1H-indazol-7-yl group, a benzofuran-4-yl group, a benzofuran-5-yl group, a benzofuran-6-yl group, or a benzofuran-7-yl group, particularly preferably a phenyl group, a 1H-indazol-5-yl group, or a benzofuran-5-yl group.

$-(R^1)n$ represents that $T^1$ is substituted with the same or different n groups of $R^1$.

n is an integer of 0 to 3.

$R^1$ is a hydrogen atom, a halogen atom, a $C_{1-4}$ alkyl group, a $C_{2-6}$ alkenyl group, a $C_{2-6}$ alkynyl group, or a $C_{1-4}$ alkoxy group, preferably a hydrogen atom, a fluorine atom, a chlorine atom, a bromine atom, a methyl group, an ethyl group, a vinyl group, an allyl group, an ethynyl group, a 1-propynyl group, a 2-propynyl group, a methoxy group, or an ethoxy group, more preferably a hydrogen atom, a fluorine atom, a chlorine atom, a bromine atom, a methyl group, a vinyl group, an ethynyl group, or a methoxy group.

In the preferred group of the following Formula (II) in the Formula (I),

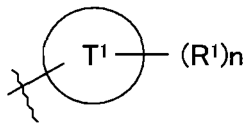

(II)

is the group of the following Formula (IIa),

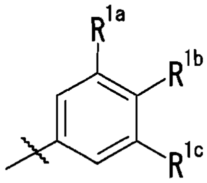

(IIa)

wherein $R^{1a}$, $R^{1b}$, and $R^{1c}$ are each the same or different and each is a hydrogen atom, a halogen atom, a $C_{1-4}$ alkyl group, a $C_{2-6}$ alkenyl group, a $C_{2-6}$ alkynyl group, or a $C_{1-4}$ alkoxy group,
or a benzofuranyl group.

In the preferred combinations of $R^{1a}$ $R^{1b}$, and $R^{1e}$, $R^{1a}$ is a halogen atom, a $C_{1-4}$ alkyl group, a $C_{2-6}$ alkenyl group, a $C_{2-6}$ alkynyl group, or a $C_{1-4}$ alkoxy group, and $R^{1b}$ and $R^{1c}$ are each a hydrogen atom. More preferably, $R^{1a}$ is a fluorine atom, a chlorine atom, a bromine atom, a methyl group, an ethyl group, a vinyl group, an allyl group, an ethynyl group, a 1-propynyl group, a 2-propynyl group, a methoxy group, or an ethoxy group, and $R^{1b}$ and $R^{1c}$ are each a hydrogen atom. Particularly preferably, $R^{1a}$ is a bromine atom, a vinyl group, an ethynyl group, or a methoxy group, and $R^{1b}$ and $R^{1c}$ are each a hydrogen atom.

In other preferred combinations of $R^{1a}$, $R^{1b}$, and $R^{1c}$, $R^{1a}$ and $R^{1c}$ are each a hydrogen atom, and $R^{1b}$ is a halogen atom, a $C_{1-4}$ alkyl group, a $C_{2-6}$ alkenyl group, a $C_{2-6}$ alkynyl group, or a $C_{1-4}$ alkoxy group. More preferably, $R^{1a}$ and $R^{1c}$ are each a hydrogen atom, and $R^{1b}$ is a fluorine atom, a chlorine atom, a bromine atom, a methyl group, an ethyl group, a vinyl group, an allyl group, an ethynyl group, a 1-propynyl group, a 2-propynyl group, a methoxy group, or an ethoxy group. Particularly preferably, $R^{1a}$ and $R^{1c}$ are each a hydrogen atom, and $R^{1b}$ is a chlorine atom or a methoxy group.

In other preferred combinations of $R^{1a}$, $R^{1b}$, and $R^{1c}$, $R^{1a}$ and $R^{1b}$ are each the same or different and each is a halogen atom, a $C_{1-4}$ alkyl group, a $C_{2-6}$ alkenyl group, a $C_{2-6}$ alkynyl group, or a $C_{1-4}$ alkoxy group, and $R^{1c}$ is a hydrogen atom. More preferably, $R^{1a}$ and $R^{1b}$ are each the same or different and each is a fluorine atom, a chlorine atom, a bromine atom, a methyl group, an ethyl group, a vinyl group, an allyl group, an ethynyl group, a 1-propynyl group, a 2-propynyl group, a methoxy group, or an ethoxy group, and $R^{1c}$ is a hydrogen atom. Particularly preferably, $R^{1a}$ and $R^{1b}$ are each the same or different and each is a fluorine atom or a chlorine atom, and $R^{1c}$ is a hydrogen atom.

More preferably, the group of the Formula (II) is a 4-chlorophenyl group, a 4-methoxyphenyl group, a 3-ethynylphenyl group, a 3-vinylphenyl group, a 3-chloro-4-fluorophenyl group, or a benzofuran-5-yl group.

$-(R^2)m$ represents that $T^2$ is substituted with the same or different m groups of $R^2$.

m is an integer of 0 to 3, preferably an integer of 0 to 2.

$R^2$ is a hydrogen atom, a hydroxy group, or a $C_{1-4}$ alkyl group, wherein said $C_{1-4}$ alkyl group may be substituted with one or two, preferably one, $C_{1-4}$ alkoxy group. Preferably, $R^2$ is a hydrogen atom, a hydroxy group, a methyl group, an ethyl group, a methoxy group, an ethoxy group, a methoxymethyl group, an ethoxymethyl group, or a methoxyethyl group, particularly preferably a hydrogen atom, a hydroxy group, a methyl group, or a methoxymethyl group.

$R^3$ is a hydrogen atom, a $C_{1-4}$ alkyl group, or a $C_{2-6}$ alkynyl group, preferably a hydrogen atom, a methyl group, an ethyl group, or a 2-propynyl group, more preferably a hydrogen atom.

In the preferred group of the following Formula (III) in the Formula (I),

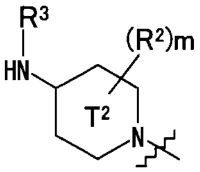

(III)

is the group of the following Formula (IIIa),

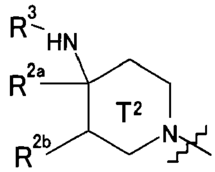

(IIIa)

wherein $R^{2a}$ and $R^{2b}$ are each the same or different and each is a hydrogen atom, a hydroxy group, or a $C_{1-4}$ alkyl group, wherein said $C_{1-4}$ alkyl group may be substituted with one or two, preferably one $C_{1-4}$ alkoxy group. Preferably, $R^{2a}$ and $R^{2b}$ are each the same or different and each is a hydrogen atom or a methyl group.

$R^3$ is the same as the above.

In the more preferred group of the Formula (III) is any one group selected from the following (IIIb) group,

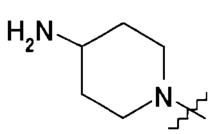

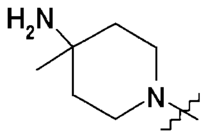

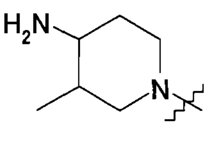

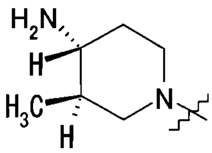

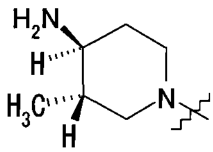

(IIIb)

.

In the preferred combinations of the groups of the Formula (II) and the Formula (III), when the group of the Formula (II) is the group of the Formula (IIa) or a benzofuranyl group, then the group of the Formula (III) is the group of the Formula (IIIa). In the more preferred combinations of the groups of the Formula (II) and the Formula (III), when the group of the Formula (II) is a 4-chlorophenyl group, a 4-methoxyphenyl group, a 3-ethynylphenyl group, a 3-vinylphenyl group, a 3-chloro-4-fluorophenyl group, or a benzofuran-5-yl group, then the group of the Formula (III) is any one group selected from the (IIIb) group.

Furthermore, it is preferred that a compound of the Formula (I) is any one compound described in the Examples.

In the more preferred compound of the Formula (I) is any one compound of the following (IV) group,

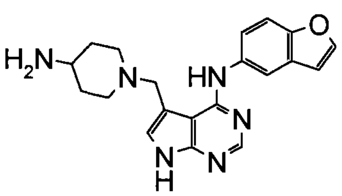

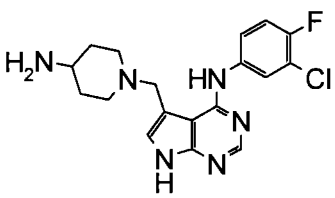

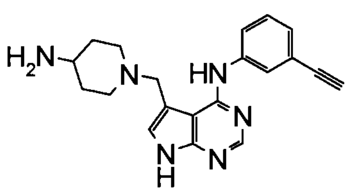

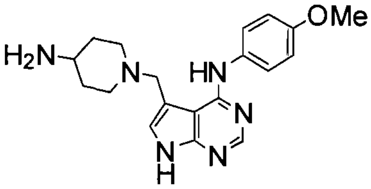 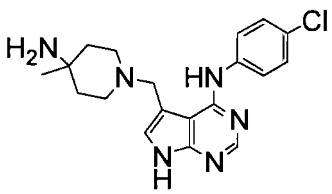 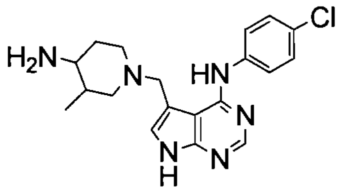

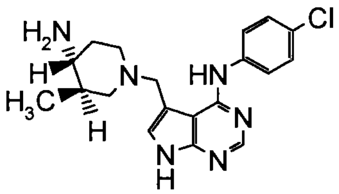 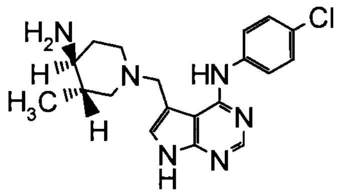 (IV)

.

In the present invention, a compound of the Formula (I) can be, if desired, converted to a pharmacologically acceptable salt according to a conventional method.

Such "pharmacologically acceptable salt" may be, for example, a salt of an inorganic acid such as hydrochloride, hydrobromide, sulfate, nitrate, phosphate or the like; a salt of a carboxylic acid such as acetate, fumarate, maleate, oxalate, malonate, succinate, citrate, malate or the like; a salt of a sulfonic acid such as methanesulfonate, ethanesulfonate, benzenesulfonate, toluenesulfonate or the like; and a salt of an amino acid such as glutamate, aspartate or the like.

A compound of the Formula (I) of the present invention or a pharmacologically acceptable salt thereof may be attached with adsorbed water or coverted into a hydrate when left in the atmosphere or recrystallized, and such hydrate is also included in the present invention.

A compound of the Formula (I) of the present invention or a pharmacologically acceptable salt thereof may be converted into a solvate when left in a solvent or recrystallized, and such solvate is also included in the present invention.

A compound of the Formula (I) of the present invention may have various isomers, for example, geometric isomers such as cis isomer, trans isomer or the like, tautomers, or optical isomers such as d-isomer, 1-isomer or the like depending on the type of the substituents or the combinations thereof, and, unless otherwise specified, a compound of the present invention also includes all of these isomers and a mixture of these isomers in any ratio.

A compound of the Formula (I) of the present invention may comprise isotopes in unnatural ratios in one or more constituent atoms. An isotope may be, for example, deuterium (2H), tritium (3H), iodine-125 (1251), carbon-14 (14C) or the like. These compounds are useful as agents for treatment or prevention, reagents for research such as assay reagents, and diagnostic agents such as diagnostic agents for *in vivo* imaging. All of the isotope variants of a compound of the Formula (I) of the present invention are, regardless of whether they are radioisotope variants or not, included in the present invention.

Furthermore, the present invention also includes a "pharmaceutically acceptable prodrug compound", which is converted into an active ingredient of a pharmaceutical composition of the present invention, a compound of the Formula (I), by the reaction with an enzyme, gastric acid or the like under physiological conditions *in vivo,* i.e., converted into the compound (I) by enzymatic oxidation, reduction, hydrolysis or the like, or converted into the compound (I) by hydrolysis or the like with gastric acid or the like.

When an amino group is present in the compound (I), the prodrug may be a compound obtained by subjecting said amino group to acylation, alkylation, phosphorylation (for example, a compound obtained by subjecting said amino group to eicosanoylation, alanylation, pentylaminocarbonylation, (5-methyl-2-oxo-1,3-dioxolen-4-yl)methoxycarbonylation, tetrahydrofuranylation, pyrrolidylmethylation, pivaloyloxymethylation, or tert-butylation, etc.) or the like, or when a hydroxy group is present in the compound (I), the prodrug may be a compound obtained by subjecting said hydroxy group to acylation, alkylation, phosphorylation, boration (for example, a compound obtained by subjecting said hydroxy group to acetylation, palmitoylation, propanoylation, pivaloylation, succinylation, fumarylation, alanylation, or dimethylaminomethylcarbonylation, etc.) or the like.

A prodrug of a compound of the present invention can be prepared from the compound (I) according to a known method. Also, a prodrug of a compound of the present invention also include one that is converted into the compound (1) under physiological conditions such as that described in "IYAKUHIN no KAIHATSU (Development of Pharmaceuticals)", Vol. 7, Design of Molecules, p.163-198, Published by HIROKAWA SHOTEN (1990).

A compound of the General Formula (I) is prepared according to, for example, the following processes. Also, the specific process for preparation of the individual compound of the present invention is demonstrated in detail in the Examples described below. Furthermore, unless otherwise specified, Et represents an ethyl group and Boc represents a tert-butoxycarbonyl group in the chemical formulas.

[Process 1]

The "Process 1" is a process for generally preparing the synthetic intermediate (6) of a pyrrolopyrimidine compound of the present invention,

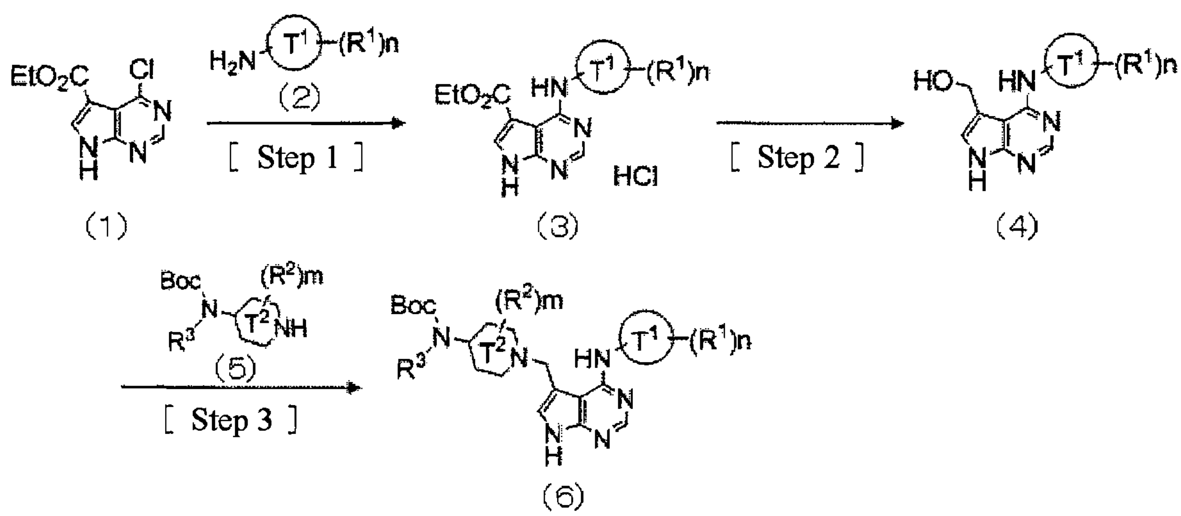


wherein $R^1$, $R^2$, $R^3$, $T^1$, $T^2$, m and n are as described above.

In the step 1, the 4-chloro-7H-pyrrolo[2,3-d]pyrimidine-5-carboxylic acid ethyl ester (1) is reacted with the aniline compound (2) in an organic solvent to prepare the compound (3).

The aniline compound (2) is a known compound, or can be prepared from a known compound according to a known process.

The amount of the aniline compound (2) used is usually 1 to 10-fold mole, preferably 1 to 3-fold mole relative to the amount of the compound (1).

The solvent used is one which does not interfere with the reaction and can dissolve the source material to some extent, and may be, for example, but is not limited to, an alcohol such as methanol, ethanol, isopropanol, or butanol; a halogenated hydrocarbon such as methylene chloride, or chloroform; an aromatic hydrocarbon such as benzene, toluene, xylene or mesitylene; an aliphatic hydrocarbon such as pentane, hexane or cyclohexane; an ether such as tetrahydrofuran, diethyl ether, diisopropyl ether, ethylene glycol dimethyl ether, 1,4-dioxane, or diglyme; or a mixture of these solvents, and preferably an alcohol, particularly preferably isopropanol.

The reaction temperature may be varied depending on the source compound, reagent, solvent or the like, and usually 0 °C to 150 °C, preferably 50 °C to 100 °C.

The reaction time may be varied depending on the reaction temperature, and usually 1 hour to 24 hours, preferably 1 hour to 3 hours.

In the step 2, the compound (3) is reduced in an organic solvent to prepare the compound (4).

The reducing agent used may be varied depending on the source compound and may be, for example, an aluminum hydride such as lithium aluminum hydride, or tri-tert-butoxy aluminum hydride, a borohydride such as sodium tetrahydroborate, or sodium tri-sec-butyl hydroborate or a metal complex thereof, preferably lithium aluminum hydride.

The amount of the reducing agent used may be varied depending on the reducing agent used or the like, and usually 1 to 10-fold mole, preferably 1 to 3-fold mole relative to the amount of the compound (3).

The solvent used may be varied depending on the source compound, reagent or the like, and is one which does not interfere with the reaction and can dissolve the source material to some extent, and may be, for example, but is not limited to, an alcohol such as methanol, or ethanol; an aromatic hydrocarbon such as benzene, toluene, xylene or mesitylene; an aliphatic hydrocarbon such as pentane, hexane or cyclohexane; an ether such as tetrahydrofuran, diethyl ether, diisopropyl ether, ethylene glycol dimethyl ether, 1,4-dioxane or diglyme; or a mixture of these solvent, preferably an ether, particularly preferably tetrahydrofuran.

The reaction temperature may be varied depending on the source compound, reagent, solvent or the like, and usually 0 °C to 150 °C, preferably 20 °C to 100 °C.

The reaction time may be varied depending on the reaction temperature, and usually 1 hour to 24 hours, preferably 1 hour to 6 hours.

In the step 3, the hydroxymethyl group at the 5-position of the compound (4) is halogenated, and then the

compound (4) is reacted with the piperidine compound (5) in an organic solvent in the presence of a base to prepare the compound (6).

The halogenating agent may be, for example, a chlorinating agent such as thionyl chloride or oxalyl chloride, or a brominating agent such as thionyl bromide or oxalyl bromide, preferably thionyl chloride.

The amount of the halogenating agent used may be varied depending on the source compound, halogenating agent used or the like, and usually 1 to 1000-fold mole, preferably 1 to 30-fold mole relative to the amount of the compound (4).

The solvent used at the halogenation is one which does not interfere with the reaction and can dissolve the source material to some extent, and may be, for example, but is not limited to, a halogenated hydrocarbon such as methylene chloride, or chloroform; an aromatic hydrocarbon such as benzene, toluene, xylene or mesitylene; an aliphatic hydrocarbon such as pentane, hexane or cyclohexane; an ether such as tetrahydrofuran, diethyl ether, diisopropyl ether, ethylene glycol dimethyl ether, 1,4-dioxane or diglyme; or a mixture of these solvent, or the halogenation can be carried out in a solvent-free condition. Preferably, the solvent is an aromatic hydrocarbon, particularly preferably toluene.

The reaction temperature at the halogenation may be varied depending on the source compound, reagent, solvent or the like, and usually 0 °C to 100 °C, preferably 0 °C to 50 °C.

The reaction time may be varied depending on the reaction temperature, and usually 1 hour to 48 hours, preferably 2 hours to 24 hours.

The piperidine compound (5) is a known compound, or can be prepared from a known compound according to a known method.

The amount of the piperidine compound (5) used is usually 1 to 10-fold mole, preferably 1 to 3-fold mole relative to the amount of the compound (4).

The base used may be, for example, an organic base such as triethylamine, pyridine, 1,8-diazabicyclo[5.4.0]-7-undecene, or diisopropylethylamine; an inorganic base such as potassium carbonate, sodium carbonate, sodium hydrogen carbonate, potassium hydroxide or sodium hydroxide, preferably triethylamine.

The amount of the base used is usually 1 to 30-fold mole, preferably 2 to 10-fold mole relative to the amount of the compound (4).

The solvent used is one which does not interfere with the reaction and can dissolve the source material to some extent, and may be, for example, but is not limited to, a nitrile such as acetonitrile, or propionitrile; a halogenated hydrocarbon such as methylene chloride, or chloroform; an aromatic hydrocarbon such as benzene, toluene, xylene or mesitylene; an aliphatic hydrocarbon such as pentane, hexane or cyclohexane; an ether such as tetrahydrofuran, diethyl ether, diisopropyl ether, ethylene glycol dimethyl ether, 1,4-dioxane or diglyme; or a mixture of these solvent, preferably a nitrile, particularly preferably acetonitrile.

The reaction temperature may be varied depending on the source compound, reagent, solvent or the like, and usually 0 °C to 150 °C, preferably 50 °C to 100 °C.

The reaction time may be varied depending on the reaction temperature, and usually 1 hour to 24 hours, preferably 1 hour to 3 hours.

[Process 2]

The "Process 2" is an alternative process for generally preparing the synthetic intermediate (6) of the pyrrolopyrimidine compound of the present invention,

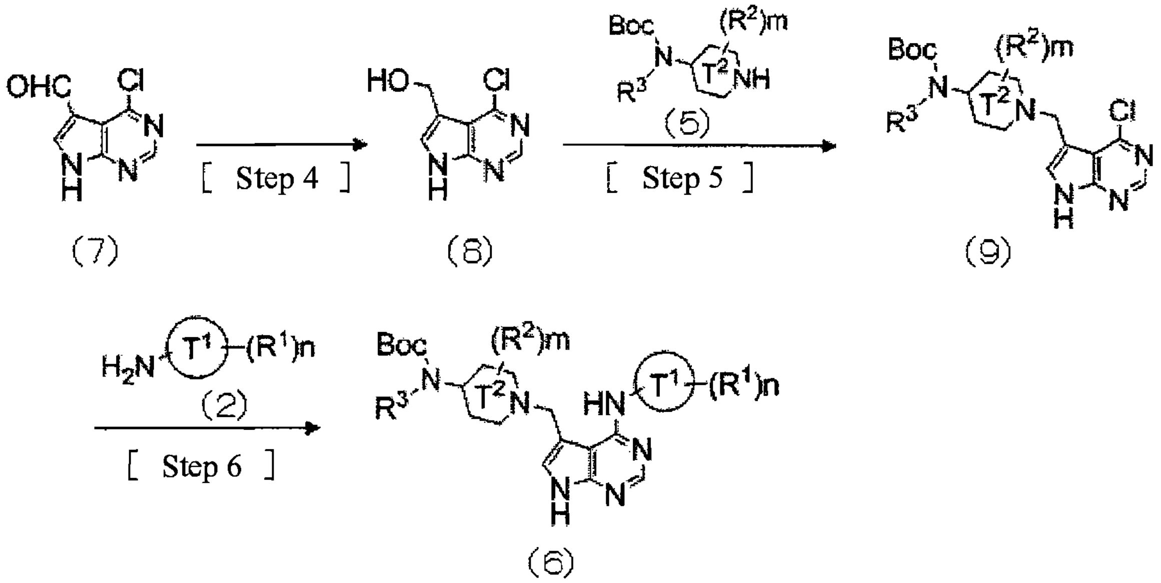


(7) (8) (9) (6)

wherein $R^1$, $R^2$, $R^3$, $T^1$, $T^2$, m and n are as described above.
In the step 4, the 4-chloro-7H-pyrrolo[2,3-d]pyrimidine-5-carbaldehyde (7) is reduced in an organic solvent to prepare the compound (8).

The compound (7) can be prepared according to, for example, the process described in WO 09/016132 pamphlet.

The reducing agent used may be, for example, an aluminum hydride such as lithium aluminum hydride, or tri-tert-butoxy aluminum hydride, a borohydride such as sodium tetrahydroborate, or sodium tri-sec-butyl hydroborate or a metal complex thereof, preferably sodium tetrahydroborate.

The amount of the reducing agent used may be varied depending on the reducing agent used or the like, and usually 0.5 to 10-fold mole, preferably 0.5 to 3-fold mole relative to the amount of the compound (7).

The solvent used may be varied depending on the reagent or the like and one which does not interfere with the reaction and can dissolve the source material to some extent, and may be, for example, but is not limited to, an alcohol such as methanol, or ethanol; an aromatic hydrocarbon such as benzene, toluene, xylene or mesitylene; an aliphatic hydrocarbon such as pentane, hexane or cyclohexane; an ether such as tetrahydrofuran, diethyl ether, diisopropyl ether, ethylene glycol dimethyl ether, 1,4-dioxane or diglyme; or a mixture of these solvent, preferably an alcohol, particularly preferably ethanol.

The reaction temperature may be varied depending on the reagent, solvent or the like, and usually 0 °C to 100 °C, preferably 0 °C to 30 °C.

The reaction time may be varied depending on the reaction temperature, and usually 30 minutes to 24 hours, preferably 30 minutes to 1 hour.

In the step 5, the hydroxymethyl group at the 5-position of the compound (8) is halogenated, and then the halogenated compound is reacted with the piperidine compound (5) in an organic solvent in the presence of a base to prepare the compound (9).

The halogenating agent may be, for example, a chlorinating agent such as thionyl chloride or oxalyl chloride, or a brominating agent such as thionyl bromide or oxalyl bromide, preferably thionyl chloride.

The amount of the halogenating agent used may be varied depending on the source compound, the halogenating agent used or the like, and usually 1 to 1000-fold mole, preferably 1 to 30-fold mole relative to the amount of the compound (8).

The solvent used at the halogenation is one which does not interfere with the reaction and can dissolve the source material to some extent, and may be, for example, but is not limited to, a halogenated hydrocarbon such as methylene chloride, or chloroform; an aromatic hydrocarbon such as benzene, toluene, xylene or mesitylene; an aliphatic hydrocarbon such as pentane, hexane or cyclohexane; an ether such as tetrahydrofuran, diethyl ether, diisopropyl ether, ethylene glycol dimethyl ether, 1,4-dioxane or diglyme; or a mixture of these solvent, or the halogenation can be carried out in a solvent-free condition. Preferably, the solvent is an ether, particularly preferably tetrahydrofuran.

The reaction temperature at the halogenation may be varied depending on the source compound, reagent, solvent or the like, and usually 0 °C to 100 °C, preferably 20 °C to 70 °C.

The reaction time may be varied depending on the reaction temperature, and usually 1 hour to 24 hours, preferably 1 hour to 3 hours.

The amount of the piperidine compound (5) used is usually 1 to 10-fold mole, preferably 1 to 2-fold mole relative to the amount of the compound (8).

The base used may be, for example, an organic base such as triethylamine, pyridine, 1,8-diazabicyclo[5.4.0]-7-undecene or diisopropylethylamine; or an inorganic base such as potassium carbonate, sodium carbonate, sodium hydrogen carbonate, potassium hydroxide or sodium hydroxide, preferably triethylamine.

The amount of the base used is usually 1 to 30-fold mole, preferably 2 to 10-fold mole relative to the amount of the compound (8).

The solvent used is one which does not interfere with the reaction and can dissolve the source material to some extent, and may be, for example, but is not limited to, a nitrile such as acetonitrile, or propionitrile; a halogenated hydrocarbon such as methylene chloride, or chloroform; an aromatic hydrocarbon such as benzene, toluene, xylene or mesitylene; an aliphatic hydrocarbon such as pentane, hexane or cyclohexane; an ether such as tetrahydrofuran, diethyl ether, diisopropyl ether, ethylene glycol dimethyl ether, 1,4-dioxane or diglyme; or a mixture of these solvent, preferably a mixed solvent of chloroform and tetrahydrofuran.

The reaction temperature may be varied depending on the source compound, reagent, solvent or the like, and usually 0 °C to 100 °C, preferably 0 °C to 50 °C.

The reaction time may be varied depending on the reaction temperature, and usually 5 minutes to 24 hours, preferably 5 minutes to 3 hours.

In the step 6, the compound (9) is reacted with the aniline compound (2) in the presence of an acid in a solvent-free condition or in an organic solvent to prepare the compound (6).

The amount of the aniline compound (2) used is usually 1 to 100-fold mole, preferably 2 to 10-fold mole relative to the amount of the compound (9).

The acid used may be, for example, an inorganic acid such as hydrochloric acid, hydrobromic acid, hydroiodic acid, sulfuric acid or nitric acid; or a sulfonic acid such as methanesulfonic acid, benzenesulfonic acid, naphthalenesulfonic acid or camphorsulfonic acid, preferably a sulfonic acid, particularly preferably methanesulfonic acid.

The amount of the acid used is usually 1 to 3-fold mole, preferably 1-fold mole relative to the amount of the compound (9).

The solvent used is one which does not interfere with the reaction and can dissolve the source material to some extent, and may be, for example, but is not limited to, an alcohol such as methanol, or ethanol; a halogenated hydrocarbon such as methylene chloride, or chloroform; an aromatic hydrocarbon such as benzene, toluene, xylene or mesitylene; an aliphatic hydrocarbon such as pentane, hexane or cyclohexane; an ether such as tetrahydrofuran, diethyl ether, diisopropyl ether, ethylene glycol dimethyl ether, 1,4-dioxane or diglyme; or a mixture of these solvent, preferably an ether, particularly preferably 1,4-dioxane.

The reaction temperature may be varied depending on the source compound, reagent, solvent or the like, and usually 0 °C to 150 °C, preferably 50 °C to 100 °C.

The reaction time may be varied depending on the reaction temperature, and usually 1 hour to 24 hours, preferably 1 hour to 3 hours.

[Process 3]

The "Process 3" is a process for generally preparing the pyrrolopyrimidine compound of the General Formula (I) of the present invention,

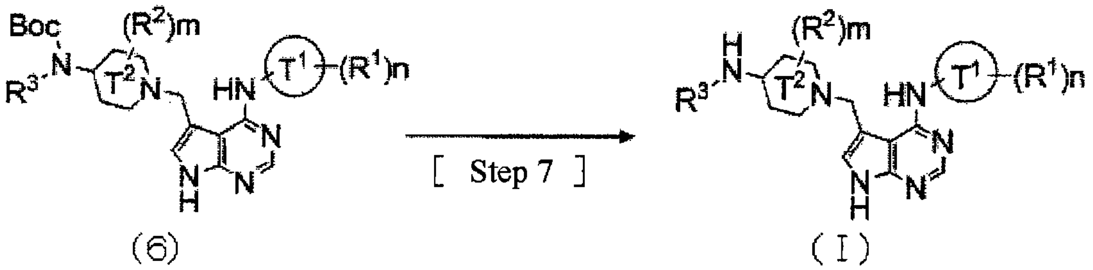


wherein $R^1$, $R^2$, $R^3$, $T^1$, $T^2$, m and n are as described above.

In the step 7, the compound (6) protected by a tert-butoxycarbonyl group is deprotected using an acid in a solvent-free condition, in an organic solvent or in water to prepare the pyrrolopyrimidine compound (I) of the present invention.

The acid used may be, for example, an inorganic acid such as hydrochloric acid, hydrobromic acid, sulfuric acid, or nitric acid, or an organic acid such as trifluoroacetic acid, methanesulfonic acid, or toluenesulfonic acid, preferably

trifluoroacetic acid.

The amount of the acid used is usually 1 to 1000-fold mole, preferably 10 to 200-fold mole relative to the amount of the compound (6).

The solvent used is one which does not interfere with the reaction and can dissolve the source material to some extent, and may be, for example, but is not limited to, water; an alcohol such as methanol, or ethanol; a halogenated hydrocarbon such as methylene chloride, or chloroform; an aromatic hydrocarbon such as benzene, toluene, xylene or mesitylene; an aliphatic hydrocarbon such as pentane, hexane or cyclohexane; an ether such as tetrahydrofuran, diethyl ether, diisopropyl ether, ethylene glycol dimethyl ether, 1,4-dioxane or diglyme; or a mixture of these solvent, preferably a halogenated hydrocarbon, particularly preferably methylene chloride.

The reaction temperature may be varied depending on the source compound, reagent, solvent or the like and usually -15 °C to 100 °C, preferably 0 °C to 50 °C.

The reaction time may be varied depending on the reaction temperature, and usually 1 hour to 24 hours, preferably 1 hour to 3 hours.

[Process 4]

The "Process 4" is an alternative process for generally preparing the pyrrolopyrimidine compound (Ia) of the present invention, wherein $R_3$ is hydrogen in the General Formula (I),

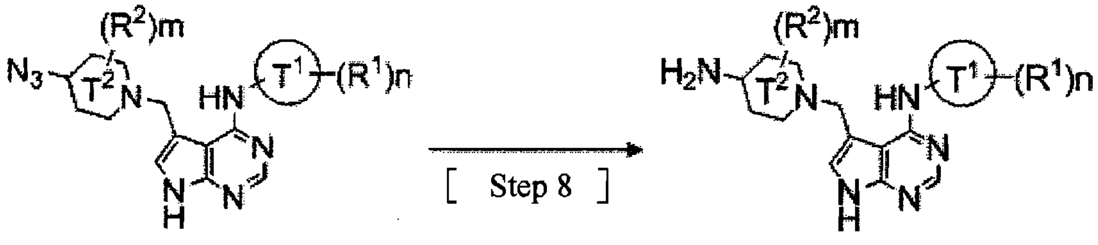


(10) (Ia)

wherein $R^1$, $R^2$, $T^1$, $T^2$, m and n are as described above.

In the step 8, the azide compound (10) is reduced using triphenylphosphine and water in an organic solvent to prepare the pyrrolopyrimidine compound (Ia) of the present invention.

The amount of triphenylphosphine used is usually 1 to 10-fold mole, preferably 1 to 3-fold mole relative to the amount of the compound (10).

The amount of water used is usually 1 to 100-fold mole, preferably 3 to 10-fold mole relative to the amount of the compound (10).

The solvent used is one which does not interfere with the reaction and can dissolve the source material to some extent, and may be, for example, but is not limited to, an ether such as tetrahydrofuran, diethyl ether, diisopropyl ether, ethylene glycol dimethyl ether, 1,4-dioxane or diglyme, preferably tetrahydrofuran.

The reaction temperature may be varied depending on the source compound, reagent, solvent or the like, and usually 0 °C to 100 °C, preferably 20 °C to 70 °C.

The reaction time may be varied depending on the reaction temperature, and usually 1 hour to 24 hours, preferably 1 hour to 3 hours.

[Process 5]

The "Process 5" is an alternative process for generally preparing the pyrrolopyrimidine compound (I) of the present invention,

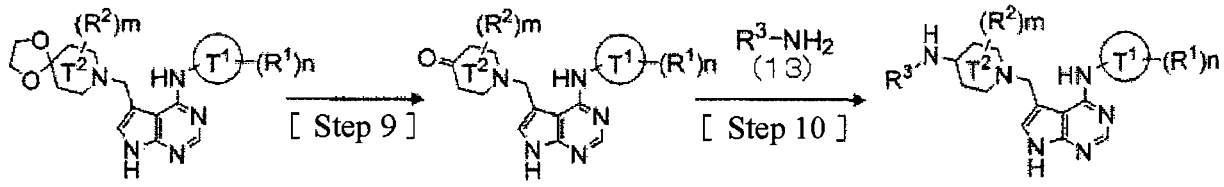


(11) (12) (I)

wherein $R^1$, $R^2$, $R^3$, $T^1$, $T^2$, m and n are as described above.
In the step 9, the 1,3-dioxolane compound (11) is deprotected in an organic solvent or water using an acid to prepare the ketone compound (12).

The 1,3-dioxolane compound (11) can be prepared according to the "Process 2" using a known compound in place of the piperidine compound (5).

The acid used may be, for example, an inorganic acid such as hydrochloric acid, hydrobromic acid, sulfuric acid, or nitric acid, or an organic acid such as trifluoroacetic acid, methanesulfonic acid, or toluenesulfonic acid, preferably hydrochloric acid.

The amount of the acid used is usually 1 to 1000-fold mole, preferably 3 to 30-fold mole relative to the amount of the compound (11).

The solvent used is one which does not interfere with the reaction and can dissolve the source material to some extent, and may be, for example, but is not limited to, water; an alcohol such as methanol, or ethanol; an ether such as tetrahydrofuran, diethyl ether, diisopropyl ether, ethylene glycol dimethyl ether, 1,4-dioxane or diglyme; a ketone such as acetone, or methyl ethyl ketone; a halogenated hydrocarbon such as methylene chloride, or chloroform; an aromatic hydrocarbon such as benzene, toluene, xylene or mesitylene; an aliphatic hydrocarbon such as pentane, hexane or cyclohexane; or a mixture of these solvent, preferably a mixed solvent of water, tetrahydrofuran and acetone.

The reaction temperature may be varied depending on the source compound, reagent, solvent or the like, and usually 0 °C to 150 °C, preferably 20 °C to 80 °C.

The reaction time may be varied depending on the reaction temperature, and usually 1 hour to 48 hours, preferably 1 hour to 24 hours.

In the step 10, the ketone compound (12) is reacted with the amino compound (13) in water or an organic solvent, and reduced with sodium triacetoxyhydroborate to prepare the pyrrolopyrimidine compound (I) of the present invention.

The amino compound (13) is a known compound, or can be prepared from a known compound according to a known method.

The amount of the amino compound (13) used is usually 1 to 10-fold mole, preferably 1 to 3-fold mole relative to the amount of the compound (12).

The amount of sodium triacetoxyhydroborate used is usually 1 to 10-fold mole, preferably 1 to 3-fold mole relative to the amount of the compound (12).

The solvent used is one which does not interfere with the reaction and can dissolve the source material to some extent, and may be, for example, but is not limited to, water; an ether such as tetrahydrofuran, diethyl ether, diisopropyl ether, ethylene glycol dimethyl ether, 1,4-dioxane or diglyme; an alcohol such as methanol, ethanol, or propanol; or a mixture of these solvent, preferably tetrahydrofuran.

The reaction temperature may be varied depending on the source compound, reagent, solvent or the like, and usually -20 °C to 100 °C, preferably 0 °C to 50 °C.

The reaction time may be varied depending on the reaction temperature, and usually 1 hour to 48 hours, preferably 2 hours to 24 hours.

After the completion of the above each reaction, the target compound is collected from the reaction mixture according to a conventional method. For example, the target compound can be obtained by neutralizing appropriately the reaction mixture, and when an insoluble matter is present, removing the matter by filtration, and then adding a water-nonmiscible organic solvent such as ethyl acetate, washing with water, and then separating the organic layer comprising the target compound, drying over a drying agent such as anhydrous magnesium sulfate, and then distilling away the solvent.

The obtained target compound can be, if necessary, separated and purified by combining appropriately conventional methods, for example, recrystallization, reprecipitation, or a common method used conventionally in separating and purifying an organic compound (for example, adsorption column chromatography method using a carrier such as silica gel or alumina, ion-exchange chromatography method, or normal-phase or reverse-phase column chromatography method on silica gel or alkylated silica gel (preferably high performance liquid chromatography method)), or chiral column chromatography method by an optically active carrier, and eluting the compound with an appropriate eluent.

When used as a therapeutic agent or a preventing agent described above, a compound of the Formula (I) of the present invention or a pharmacologically acceptable salt thereof can be administered alone or in an appropriate mixture with a pharmacologically acceptable excipient, diluent or the like, for example, orally in the form of tablet, capsule, granule, powder, syrup or the like, or parenterally in the form of injection, suppository or the like.

These formulations can be prepared according to a known method using additives, such as an excipient (which may be, e.g., an organic excipient, for example, a sugar derivative such as lactose, saccharose, glucose, mannitol, or sorbitol; a starch derivative such as corn starch, potato starch, alpha starch, or dextrin; a cellulose derivative such as crystalline cellulose; gum arabic; dextran; or pullulan: and an inorganic excipient, for example, a silicate derivative such as light anhydrous silicic acid, synthetic aluminum silicate, calcium silicate, or magnesium aluminometasilicate; a phosphate such as calcium hydrogen phosphate; a carbonate such as calcium carbonate; a sulfate such as calcium sulfate), a lubricant (which may be, for example, stearic acid, or a metal stearate such as calcium stearate, or magnesium stearate; talc; colloid silica; a wax such as Veegum, or spermaceti; boric acid; adipic acid; a sulfate such as sodium sulfate; glycol; fumaric acid; sodium benzoate; DL leucine; a fatty acid sodium salt; a lauryl sulfate such as sodium lauryl sulfate, or magnesium lauryl sulfate; a silicic acid derivative such as anhydrous silicic acid, or silicic acid hydrate; and a starch derivative described above), a binder (which may be, for example, hydroxypropylcellulose, hydroxypropylmethylcellulose, polyvinylpyrrolidone, macrogol, and the same compounds as described above in said excipient), a disintegrant (which may be, for example, a cellulose derivative such as low substituted hydroxypropylcellulose, carboxymethylcellulose, carboxymethylcellulose calcium, or internally-cross-linked carboxymethylcellulose sodium; a chemically-modified starch or cellulose such as carboxymethyl starch, sodium carboxymethyl starch, or cross-linked polyvinylpyrrolidone), a stabilizing agent (which may be, for example, a parahydroxybenzoate ester such as methylparaben, or propylparaben; an alcohol such as chlorobutanol, benzyl alcohol, or phenylethyl alcohol; benzalkonium chloride; a phenol such as phenol, or cresol; thimerosal; dehydroacetic acid; and sorbic acid), a flavoring agent (which may be, for example, a conventionally used sweetening agent, acidulant, perfume or the like), a diluent or the like.

The dosage of a compound of the present invention may be varied widely depending on various conditions such as the activity of the agent, the disease condition, age, and body weight of a patient (warm-blooded animal, especially human) and the like, and it is desirable that the dosage per administration which ranges from 0.01 mg/kg body weight as lower limit to 5000 mg/kg body weight as upper limit in oral administration, or from 0.001 mg/kg body weight as lower limit to 5000 mg/kg body weight as upper limit in intravenous administration is administered to a patient one to several times per day depending on the disease condition. Preferred dosage is 0.1 mg/kg body weight to 100 mg/kg body weight per day.

A compound of the present invention may be used in combination with other anti-tumor agents. For example, the anti-tumor agent may be an alkylating agent, an antimetabolite, an anti-tumor antibiotic, an anti-tumor plant component, a BRM (Biological Response Modifier), a hormone, a vitamin, an anti-tumor antibody, a molecular target drug, an other anti-tumor agent or the like.

More specifically, the alkylating agent may be, for example, an alkylating agent such as nitrogen mustard, nitrogen mustard N-oxide or chlorambucil, an aziridine alkylating agent such as carboquone or thiotepa, an epoxide alkylating agent such as dibromomannitol or dibromodulcitol, a nitrosourea alkylating agent such as carmustine, lomustine, semustine, nimustine hydrochloride, streptozocin, chlorozotocin or ranimustine, busulfan, improsulfan tosilate, dacarbazine or the like.

The antimetabolite may be, for example, a purine antimetabolite such as 6-mercaptopurine, 6-thioguanine or thioinosine, a pyrimidine antimetabolite such as fluorouracil, tegafur, tegafur-uracil, carmofur, doxifluridine, broxuridine, cytarabine or enocitabine, a folic acid antimetabolite such as methotrexate or trimetrexate, or the like.

The anti-tumor antibiotic may be, for example, an anthracycline antibiotic anti-tumor agent such as mitomycin C, bleomycin, peplomycin, daunorubicin, aclarubicin, doxorubicin, pirarubicin, THP-adriamycin, 4'-epidoxorubicin or epirubicin, chromomycin A3, actinomycin D or the like.

The anti-tumor plant component may be, for example, a vinca alkaloid such as vindesine, vincristine or vinblastine, a taxane such as paclitaxel or docetaxel, an epipodophyllotoxin such as etoposide or teniposide, or the like.

The BRM may be, for example, tumor necrosis factor, indomethacin or the like.

The hormone may be, for example, hydrocortisone, dexamethasone, methylprednisolone, prednisolone, prasterone, betamethasone, triamcinolone, oxymetholone, nandrolone, methenolone, fosfestrol, ethinyl estradiol, chlormadinone, medroxyprogesterone or the like.

The vitamin may be, for example, vitamin C, vitamin A or the like.

The anti-tumor antibody or molecular target drug may be trastuzumab, rituximab, cetuximab, nimotuzumab, denosumab, bevacizumab, infliximab, imatinib mesylate, gefitinib, erlotinib, sunitinib, lapatinib, sorafenib or the like.

The other anti-tumor agent may be, for example, cisplatin, carboplatin, oxaliplatin, tamoxifen, camptothecin derivative, ifosfamide, cyclophosphamide, melphalan, L-asparaginase, aceglatone, sizofiran, Picibanil, procarbazine, pipobroman, neocarzinostatin, hydroxyurea, ubenimex, Krestin or the like.

The present invention also includes a method for preventing and/or treating cancer, characterized in that the

method comprises administering a compound of the present invention or a pharmacologically acceptable salt thereof.

Furthermore, the present invention also includes a use of a compound of the present invention or pharmacologically acceptable salt thereof for manufacturing said medicament.

EXAMPLES

Hereinafter, Examples of the compounds of the present invention, and examples of the synthetic intermediates, examples of formulations and results of pharmacological tests are shown. It should be noted that these exemplifications are provided for purpose of better understanding of the present invention, and not intended to limit the range of the present invention. Also, in the Examples and the Reference Examples, each of the [Enantiomer 1] and [Enantiomer 2] described just after a chemical name means that the single enantiomer obtained in each of the Reference Example 14-1 and the Reference Example 14-2 is utilized as an intermediate respectively. Even if a chemical formula described in an Example or a Reference Example is depicted as a planar structure, a title compound prepared from a single enantiomer is meant to be present as a single enantiomer. Also, unless otherwise specified, Bz represents a benzoyl group, Bn represents a benzyl group, and MsOH represents methanesulfonic acid in chemical formulas.

[Reference Examples]

(Reference Example 1-1)

4-(Benzofuran-5-ylamino)-7H-pyrrolo[2,3-d]pyrimidine-5-carboxylic acid ethyl ester hydrochloride

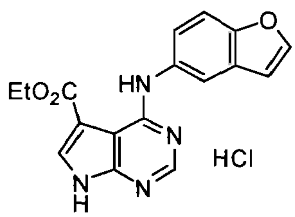

To a solution of 4-chloro-7H-pyrrolo[2,3-d]pyrimidine-5-carboxylic acid ethyl ester (0.383 g, 1.70 mmol) in isopropanol (15 ml) was added 5-aminobenzo[b]furan (0.250 g, 1.88 mmol), and the mixture was heated for 1 hour under reflux.

After the completion of the reaction, the reaction mixture was cooled to 0 °C, and then the precipitated solid was collected by filtration, washed with diethyl ether, and dried under reduced pressure to give the compound of the Reference Example 1-1 (0.528 g, 1.47 mmol, yield 87%).

Mass (EI, m/z): 322 $[M]^+$ .

$^1$H-NMR (DMSO-$d_6$) $\delta$: 12.95 (1H, br s), 11.06 (1H, s), 8.39 (1H, s), 8.24 (1H, d, J = 1.7 Hz), 8.15 (1H, s), 8.02 (1H, d, J = 2.0 Hz), 7.65 (1H, d, J = 8.8 Hz), 7.56 (1H, dd, J = 8.8, 2.0 Hz), 7.02 (1H, d, J = 1.7 Hz), 4.40 (2H, q, J = 7.1 Hz), 1.37 (3H, t, J = 7.1 Hz).

Hereinafter, the compounds of the Reference Examples 1-2 to 1-4 were prepared according to the process for preparation described in the Reference Example 1-1.

(Reference Example 1-2)

4-(3-Chloro-4-fluorophenylamino)-7H-pyrrolo[2,3-d]pyrimidine-5-carboxylic acid ethyl ester hydrochloride

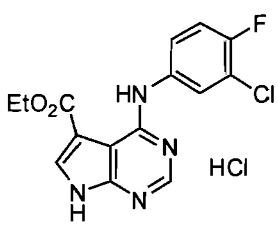

Mass (EI, m/z): 334 $[M]^+$.

$^1$H-NMR (DMSO-$d_6$) δ: 12.87 (1H, br s), 11.02 (1H, s), 8.45 (1H, s), 8.31 (1H, dd, J = 6.8, 2.7 Hz), 8.14 (1H, s), 7.58 (1H, ddd, J = 9.0, 4.2, 2.7 Hz), 7.43 (1H, t, J = 9.0 Hz), 4.39 (2H, q, J = 7.1 Hz), 1.36 (3H, t, J = 7.1 Hz).

(Reference Example 1-3)

4-(3-Vinylphenylamino)-7H-pyrrolo[2,3-d]pyrimidine-5-carboxylic acid ethyl ester hydrochloride

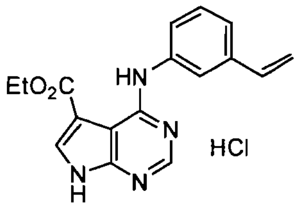

Mass (EI, m/z): 308 [M]$^+$.
$^1$H-NMR (DMSO-$d_6$) δ: 12.87 (1H, br s), 10.98 (1H, s), 8.42 (1H, s), 8.13 (1H, s), 7.88 (1H, t, J = 1.7 Hz), 7.76 (1H, ddd, J = 8.1, 2.0 Hz, 1.0 Hz), 7.38 (1H, t, J = 7.8 Hz), 7.24 (1H, dt, J = 7.7, 1.2 Hz), 6.78 (1H, dd, J = 17.7, 10.9 Hz), 5.85 (1H, dd, J = 17.7, 0.9 Hz), 5.31 (1H, dd, J = 11.0, 1.0 Hz), 4.40 (2H, q, J = 7.1 Hz), 1.37 (3H, t, J = 7.1 Hz).
(Reference Example 1-4)

4-(3-Chlorophenylamino)-7H-pyrrolo[2,3-d]pyrimidine-5-carboxylic acid ethyl ester hydrochloride

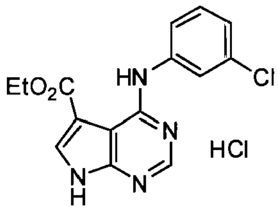

Mass (EI, m/z): 316 [M]$^+$.
$^1$H-NMR ($CDCl_3$) δ: 11.82 (1H, s), 8.54 (1H, s), 8.01 (1H, t, J = 2.1 Hz), 7.94 (1H, s), 7.68 (1H, ddd, J = 8.1, 1.0, 2.1 Hz), 7.3 5(1H, t, J = 8.1 Hz), 7.20 (1H, ddd, J = 8.0, 1.0, 2.1 Hz), 4.46 (2H, q, J = 7.2 Hz), 1.45 (3H, t, J = 7.2 Hz).

(Reference Example 2-1)

[4-(Benzofuran-5-ylamino)-7H-pyrrolo[2,3-d]pyrimidin-5-yl]methanol

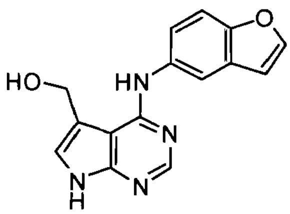

To a solution of 4-(benzofuran-5-ylamino)-7H-pyrrolo[2,3-d]pyrimidine-5-carboxylic acid ethyl ester hydrochloride (Reference Example 1-1) (0.520 g, 1.45 mmol) in tetrahydrofuran (10 ml) was added 1 M lithium aluminum hydride/ tetrahydrofuran solution (3.2 ml, 3.2 mmol) at 0°C. Then, the mixture was stirred for 2.5 hours at room temperature, followed by stirring for 1 hour at 60 °C.
After the completion of the reaction, the reaction mixture was diluted with tetrahydrofuran, cooled to 0 °C, and

then 1.3 ml of water and 0.8 ml of 2 N aqueous sodium hydroxide solution were added sequentially. The mixture was stirred for 20 minutes at room temperature, and then diluted with chloroform and methanol, and magnesium sulfate was added. The solid was filtered out using a Celite (trade name) filter, and the filtrate was concentrated under reduced pressure. The obtained residue was suspended in a mixed solution of chloroform and diethyl ether (1:1 (V/V)), collected by filtration, and dried under reduced pressure to give the compound of the Reference Example 2-1 (0.384 g, 1.37 mmol, yield 94%). Mass (EI, m/z): 280 $[M]^+$.
$^1$H-NMR (DMSO-$d_6$) δ: 11.59 (1H, br s), 10.03 (1H, s), 8.29 (1H, s), 8.24 (1H, d, J = 1.7 Hz), 7.96 (1H, d, J = 2.0 Hz), 7.57 (1H, d, J = 9.0 Hz), 7.48 (1H, dd, J = 9.0, 2.0 Hz), 7.16 (1H, s), 6.98 (1H, d, J = 1.7 Hz), 6.43 (1H, t, J = 3.9 Hz), 4.77 (2H, d, J = 3.9 Hz).
Hereinafter, the compounds of the Reference Examples 2-2 to 2-4 were prepared according to the process for preparation described in the Reference Example 2-1.

(Reference Example 2-2)

[4-(3-Chloro-4-fluorophenylamino)-7H-pyrrolo[2,3-d]pyrimidin-5-yl]methanol

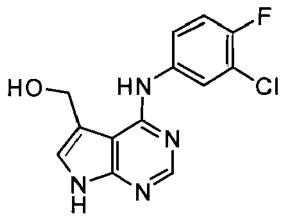

Mass (EI, m/z): 292 $[M]^+$.
$^1$H-NMR (DMSO-$d_6$) δ: 11.69 (1H, br s), 10.18 (1H, s), 8.34 1H, s), 8.25 (1H, dd, J = 6.8, 2.7 Hz), 7.56 (1H, ddd, J = 9.0, 4.4, 2.7 Hz), 7.40 (1H, t, J = 9.0 Hz), 7.20 (1H, s), 6.48 (1H, br s), 4.76 (2H, s).

(Reference Example 2-3)

[4-(3-Vinylphenylamino)-7H-pyrrolo[2,3-d]pyrimidin-5-yl]methanol

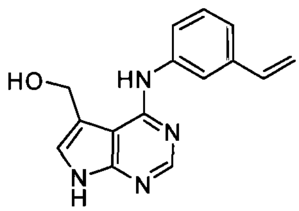

Mass (EI, m/z): 266 $[M]^+$.
$^1$H-NMR (DMSO-$d_6$) δ:11.62 (1H, br s), 10.05 (1H, s), 8.30 (1H, s), 7.81 (1H, t, J = 2.0 Hz), 7.77 (1H, ddd, J = 8.1, 2.0, 1.0 Hz), 7.32 (1H, t, J = 7.8 Hz), 7.18 (1H, s), 7.13 (1H, d, J = 7.6 Hz), 6.74 (1H, dd, J = 17.7, 10.9 Hz), 6.43 (1H, s), 5.81 (1H, dd, J = 17.7, 1.1 Hz), 5.29 (1H, dd, J = 11.0, 1.0 Hz), 4.76 (2H, s).

(Reference Example 2-4)

[4-(3-Chlorophenylamino)-7H-pyrrolo[2,3-d]pyrimidin-5-yl]methanol

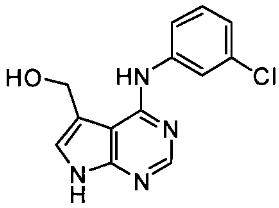

Mass (EI, m/z): 274 $[M]^+$.
$^1$H-NMR ($CDCl_3$) δ: 9.31 (1H, s), 8.43 (1H, s), 7.93 (1H, t, J = 2.0 Hz), 7.61 (1H, ddd, J = 8.1, 1.0, 2.0 Hz), 7.27 (1H, t, J = 8.1 Hz), 7.03 (1H, ddd, J = 8.1, 1.0, 2.0 Hz), 7.00 (1H, s), 4.91 (2H, s).

(Reference Example 3-1)

{1-[4-(Benzofuran-5-ylamino)-7H-pyrrolo[2,3-d]pyrimidin-5-ylmethyl]piperidin-4-yl}carbamic acid tert-butyl ester

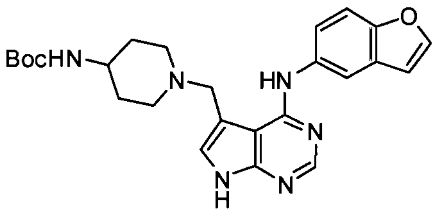

To a suspension of [4-(benzofuran-5-ylamino)-7H-pyrrolo[2,3-d]pyrimidin-5-yl]methanol (Reference Example 2-1) (0.182 g, 0.650 mmol) in toluene (6 ml) was added thionyl chloride (0.60 ml, 8.2 mmol) under nitrogen atmosphere, and the mixture was stirred for 15.5 hours at room temperature.

After the completion of the reaction, the solid was collected by filtration, washed with diethyl ether, and dried under reduced pressure.

To a suspension of the obtained solid in acetonitrile (6 ml) were added triethylamine (0.50 ml, 3.6 mmol) and 4-(tert-butoxycarbonylamino)piperidine (0.171 g, 0.854 mmol) under nitrogen atmosphere, and the mixture was heated for 1.5 hours under reflux.

After the completion of the reaction, the reaction mixture was concentrated under reduced pressure, and then subjected to silica gel column chromatography (elution solvent: chloroform:methanol = 60:1 to 50:1 (V/V)), and the fraction comprising the target compound was concentrated under reduced pressure to give the compound of the Reference Example 3-1 (0.219 g, 0.473 mmol, yield 73%).
Mass (EI, m/z): 462 $[M]^+$.
$^1$H-NMR (DMSO-$d_6$) δ:11.56 (1H, br s), 10.96 (1H, s), 8.24 (1H, d, J = 2.0 Hz), 8.23 (1H, s), 7.96 (1H, d, J = 2.0 Hz), 7.57 (1H, d, J = 8.9 Hz), 7.50 (1H, dd, J = 8.9, 2.0 Hz), 7.14 (1H, s), 6.98-6.96 (2H, m), 3.66 (2H, s), 3.52-3.31 (1H, m), 3.02 (2H, br d, J = 11.4 Hz), 2.11 (2H, br t, J = 11.4 Hz), 1.80 (2H, br d, J = 10.7 Hz), 1.53 (2H, br q, J = 10.7 Hz), 1.38 (9H, s).
Hereinafter, the compounds of the Reference Examples 3-2 to 3-6 were prepared according to the process for preparation described in the Reference Example 3-1.

(Reference Example 3-2)

{1-[4-(3-Chloro-4-fluorophenylamino)-7H-pynrrolo[2,3-d]pyrimidin-5-ylmethyl]piperidin-4-yl}carbamic acid tert-butyl ester

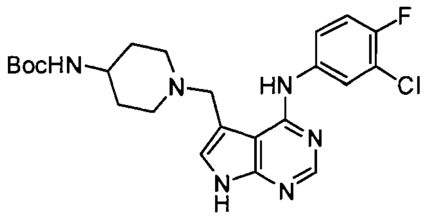

Mass (EI, m/z): 474 $[M]^+$.
$^1$H-NMR (DMSO-$d_6$) δ: 11.67 (1H, br s), 11.20 (1H, s), 8.32 (1H, dd, J = 7.0, 2.6 Hz), 8.29 (1H, s), 7.50 (1H, ddd, J = 9.0, 4.2, 2.6 Hz), 7.40 (1H, t, J = 9.0 Hz), 7.19 (1H, d, J = 1.2 Hz), 6.94 (1H, d, J = 7.6 Hz), 3.66 (2H, s), 3.38-3.24 (1H, m), 3.00 (2H, br d, J = 11.2 Hz), 2.09 (2H, br t, J = 11.2 Hz), 1.78 (2H, br d, J = 11.6 Hz), 1.45 (2H, br q, J = 11.6 Hz), 1.37 (9H, s).

(Reference Example 3-3)

{1-[4-(3-Vinylphenylamino)-7H-pyrrolo[2,3-d]pyrimidin-5-ylmethyl]piperidin-4-yl}carbamic acid tert-butyl ester

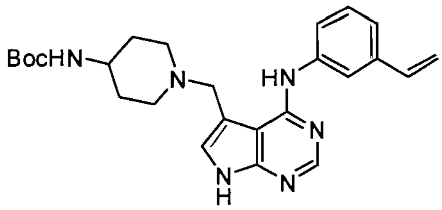

Mass (EI, m/z): 448 $[M]^+$.
$^1$H-NMR (DMSO-$d_6$) δ: 11.60 (1H, br s), 10.94 (1H, s), 8.26 (1H, s), 7.83 (1H, t, J = 1.7 Hz), 7.74 (1H, dd, J = 7.9, 1.8 Hz), 7.34 (1H, t, J = 7.9 Hz), 7.17-7.14 (2H, m), 6.99 (1H, d, J = 6.8 Hz), 6.77 (1H, dd, J = 17.8, 11.0 Hz), 5.81 (1H, dd, J = 17.7, 0.9 Hz), 5.29 (1H, dd, J = 11.0, 0.7 Hz), 3.65 (2H, s), 3.39-3.34 (1H, m), 3.01 (2H, d, J = 11.0 Hz), 2.11 (2H, t, J = 11.7 Hz), 1.81 (2H, d, J = 11.0 Hz), 1.59-1.44 (2H, m), 1.37 (9H, s).

(Reference Example 3-4)

{1-[4-(3-Chlorophenylamino)-7H-pyrrolo[2,3-d]pyrimidin-5-ylmethyl]-piperidin-4-yl}carbamic acid tert-butyl ester

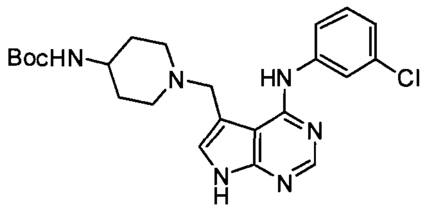

Mass (EI, m/z): 456 $[M]^+$.
$^1$H-NMR ($CDCl_3$) δ: 11.02-10.99 (1H, br m), 9.57 (1H, br s), 8.44 (1H, s), 7.94 (1H, t, J = 2.0 Hz), 7.60 (1H, ddd, J = 8.1, 1.0, 2.0 Hz), 7.29 (1H, t, J = 8.1 Hz), 7.03 (1H, ddd, J = 8.1, 1.0, 2.0 Hz), 6.96 (1H, d, J = 2.0 Hz), 4.46 (1H, d, J = 8.3 Hz), 3.69 (2H, s), 3.58 (1H, br s), 3.05 (2H, d, J = 9.8 Hz), 2.23 (2H, t, J = 11.1 Hz), 2.04 (2H, d, J = 11.2 Hz), 1.57-1.43 (11H, m).

(Reference Example 3-5)

trans-4-Azide-1-[4-(benzofuran-5-ylamino)-7H-pyrrolo[2,3-d]pyrimidin-5-ylmethyl]piperidin-3-ol

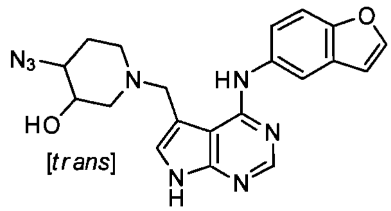

Mass (EI, m/z): 404 $[M]^+$.
$^1$H-NMR (DMSO-$d_6$) δ: 11.60 (1H, br s), 10.74 (1H, s), 8.24 (1H, s), 8.22 (1H, d, J = 1.7 Hz), 7.98 (1H, d, J = 2.0 Hz), 7.58 (1H, d, J = 8.8 Hz), 7.43 (1H, dd, J = 8.8, 2.0 Hz), 7.17 (1H, s), 6.96 (1H, d, J = 1.7 Hz), 5.45 (1H, d, J = 4.9 Hz), 3.81 (1H, d, J = 13.2 Hz), 3.65 (1H, d, J = 13.2 Hz), 3.55-3.40 (2H, m), 3.13-2.97 (2H, m), 2.16 (1H, br t, J = 11.4 Hz), 2.04-1.87 (2H, m), 1.45 (1H, qd, J = 12.0, 3.0 Hz).

(Reference Example 3-6)

trans-4-Azide-1-[4-(3-chloro-4-fluorophenylamino)-7H-pyrrolo[2,3-d]pyrimidin-5-ylmethyl]piperidin-3-ol

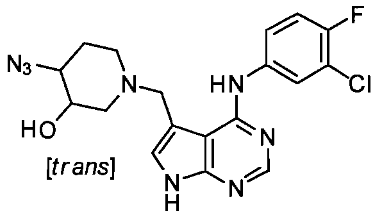

Mass (EI, m/z): 416 $[M]^+$.
$^1$H-NMR (DMSO-$d_6$) δ: 11.71 (1H, br s), 10.90 (1H, s), 8.30 (1H, s), 8.24 (1H, dd, J = 6.7, 2.4 Hz), 7.54 (1H, ddd, J = 9.0, 4.1, 2.4 Hz), 7.42 (1H, t, J = 9.0 Hz), 7.22 (1H, s), 5.43 (1H, d, J = 4.2 Hz), 3.81 (1H, d, J = 13.2 Hz), 3.63 (1H, d, J = 13.2 Hz), 3.48-3.33 (2H, m), 3.11-2.93 (2H, m), 2.18 (1H, br t, J = 11.2 Hz), 2.05-1.88 (2H, m), 1.41 (1H, br q, J = 11.2 Hz).

(Reference Example 4)

(4-Chloro-7H-pyrrolo[2,3-d]pyrimidin-5-yl)methanol

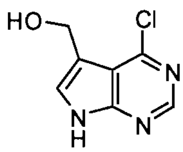

To a solution of 4-chloro-7H-pyrrolo[2,3-d]pyrimidine-5-carbaldehyde (see WO 09/016132 pamphlet) (6.75 g, 37.2 mmol) in ethanol (100 ml) was added sodium tetrahydroborate (0.704 g, 18.6 mmol), and the mixture was stirred for 20 minutes at room temperature. Then, to the mixture was added sodium tetrahydroborate (0.352 g, 9.30 mmol) and stirred for 20 minutes at room temperature.

After the completion of the reaction, the reaction mixture was concentrated under reduced pressure, and the obtained crude body was dissolved in a mixed solution of ethyl acetate/tetrahydrofuran, and washed with saturated aqueous ammonium chloride solution. The obtained organic layer was dried over anhydrous magnesium sulfate, and then concentrated under reduced pressure. The resulting solid was suspended in diisopropyl ether, collected by filtration, and dried under reduced pressure to give the compound of the Reference Example 4 (4.93 g, 26.9 mmol, yield 72%).
Mass (EI, m/z): 183 $[M]^+$.
$^1$H-NMR (DMSO-$d_6$) δ: 12.37 (1H, br s), 8.55 (1H, s), 7.55 (1H, s), 5.03 (1H, t, J = 5.1 Hz), 4.75 (2H, dd, J = 5.1, 1.0 Hz).

(Reference Example 5-1)

[1-(4-Chloro-7H-pyrrolo[2,3-d]pyrimidin-5-ylmethyl)piperidin-4-yl]-carbamic acid tert-butyl ester methanesulfonate

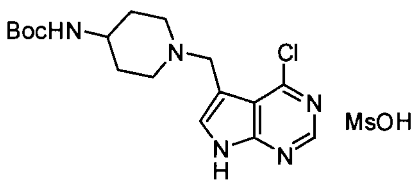

To a suspension of (4-chloro-7H-pyrrolo[2,3-d]pyrimidin-5-yl)methanol (Reference Example 4) (5.51 g, 30 mmol) and tetrahydrofuran (90 ml) was added thionyl chloride (15 ml, 207 mmol), and the mixture was heated for 2 hours under reflux condition.
After the completion of the reaction, the reaction mixture was concentrated under reduced pressure, and further dried under reduced pressure. To the obtained residue were added tetrahydrofuran (90 ml), chloroform (40 ml), and 4-(tert-butoxycarbonylamino)piperidine (6.07 g, 30 mmol), and the mixture was stirred for 5 minutes at room temperature. Then, to the mixture was added triethylamine (20.9 ml, 150 mmol), and stirred for 10 minutes at room temperature.
After the completion of the reaction, the reaction mixture was concentrated under reduced pressure, and ethyl acetate (200 ml) was added. The organic layer was washed with saturated aqueous ammonium chloride solution, dried over anhydrous magnesium sulfate, and then concentrated under reduced pressure. The obtained residue was subjected to silica gel column chromatography (elution solvent: chloroform:methanol = 19:1 (V/V)), and the fraction comprising the target compound was concentrated under reduced pressure to give the crude product (9.71 g, 26.5 mmol). The obtained crude product was dissolved in acetone (130 ml), and to the solution was added methanesulfonic acid (1.72 ml, 26.5 mmol), and then diisopropyl ether (100 ml) was added to precipitate the salt. The precipitated product was collected by filtration, and dried under reduced pressure to give the compound of the Reference Example 5-1 (3.51 g, 13.5 mmol, yield 37%).
Mass (EI, m/z): 365 $[M]^+$.
$^1$H-NMR ($CD_3$ OD) δ: 8.64 (1H, s), 7.90 (1H, s), 4.67 (2H, s), 3.68-3.31 (3H, m), 3.22 (2H, td, J = 13.2, 2.6 Hz), 2.70 (3H, s), 2.15 (2H, d, J = 14.4 Hz), 1.68 (2H, dq, J = 4.0, 13.0 Hz), 1.44 (9H, s).
Hereinafter, the compounds of the Reference Examples 5-2 to 5-6 were prepared according to the process for preparation described in the Reference Example 5-1.
Note that 4-azidepiperidin-3-ol used in the preparation of the compound of the Reference Example 5-5 was prepared with reference to WO 05/066176 pamphlet. Also, in the preparation of the compounds of the Reference Examples 5-5 and 5-6, the compounds were not in the form of salt, but in free form, because said compounds were not subjected to a reaction for being converted into methanesulfonates.

(Reference Example 5-2)

[1-(4-Chloro-7H-pyrrolo[2,3-d]pyrimidin-5-ylmethyl)-4-methylpiperidin-4-yl]carbamic acid tert-butyl ester methanesulfonate

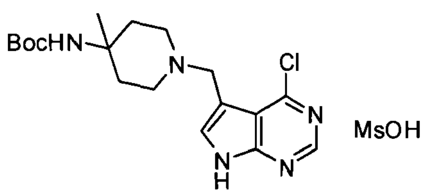

Mass (EI, m/z): 379 $[M]^+$.
$^1$H-NMR ($CD_3$ OD) δ: 8.64 (1H, s), 7.89 (1H, s), 4.69 (2H, s), 3.49 (2H, d, J = 12.7 Hz), 3.25 (2H, t, J = 13.1 Hz), 2.69 (3H, s), 2.46 (2H, d, J = 13.9 Hz), 1.67 (2H, dt, J = 4.6, 14.2 Hz), 1.44 (9H, s), 1.32 (3H, s).

(Reference Example 5-3)

[1-(4-Chloro-7H-pyrrolo[2,3-d]pyrimidin-5-ylmethyl)-4-methoxymethylpiperidin-4-yl]carbamic acid tert-butyl ester methanesulfonate

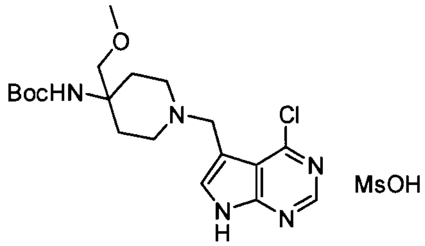

Mass (EI, m/z): 409 $[M]^+$.
$^1$H-NMR ($CD_3$ OD) δ: 8.64 (1H, s), 7.89 (1H, s), 4.69 (2H, s), 3.52 (2H, d, J = 12.0 Hz), 3.43 (2H, s), 3.33 (3H, s), 3.26 (2H, t, J = 10.6 Hz), 2.70 (3H, s), 2.40 (2H, d, J = 14.2 Hz), 1.85 (2H, td, J = 14.3, 3.8 Hz), 1.44 (9H, s).

(Reference Example 5-4)

[1-(4-Chloro-7H-pyrrolo[2,3-d]pyrimidin-5-ylmethyl)piperidin-4-yl]methylcarbamic acid tert-butyl ester methanesulfonate

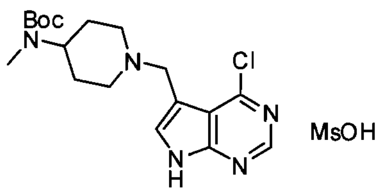

Mass (EI, m/z): 379 $[M]^+$.
$^1$H-NMR ($CDCl_3$) δ: 11.50 (1H, s), 10.38 (1H, br s), 8.43 (1H, s), 8.32 (1H, br s), 4.60 (2H, d, J = 5.4 Hz), 4.36 (1H, br s), 3.80 (2H, d, J = 11.5 Hz), 3.08-2.96 (5H, m), 2.79 (3H, s), 2.37 (2H, dq, J = 3.0, 12.8 Hz), 1.90 (2H, d, J = 12.0 Hz), 1.46 (9H, s).

(Reference Example 5-5)

trans-4-Azide-1-(4-chloro-7H-pyrrolo[2,3-d]pyrimidin-5-ylmethyl)piperidin-3-ol

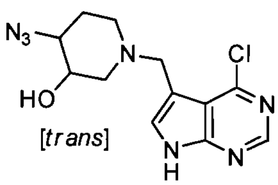

Mass (EI, m/z): 307 $[M]^+$.
$^1$H-NMR ($CD_3$ OD) δ: 8.50 (1H, s), 7.48 (1H, s), 3.89 (1H, dd, J = 13.7, 0.6 Hz), 3.83 (1H, dd, J = 13.7, 0.6 Hz), 3.50 (1H, td, J = 9.5, 4.5 Hz), 3.21-3.07 (2H, m), 2.99-2.93 (1H, br m), 2.16 (1H, td, J = 11.8, 2.6 Hz), 2.02 (1H, dd, J = 10.9, 10.1 Hz), 1.93 (1H, ddt, J = 12.5, 4.6, 2.6 Hz), 1.49 (1H, tdd, J = 12.5, 12.0, 4.6 Hz).

(Reference Example 5-6)

8-(4-Chloro-7H-pyrrolo[2,3-d]pyrimidin-5-ylmethyl)-1,4-dioxa-8-azaspiro[4.5]decane

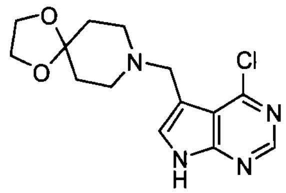

Mass (EI, m/z): 308 $[M]^+$.
$^1$H-NMR ($CDCl_3$) δ: 10.19 (1H, br s), 8.63 (1H, s), 7.32 (1H, s), 3.96 (4H, s), 3.88 (2H, d, J = 0.7 Hz), 2.66 (4H, t, J = 5.5 Hz), 1.76 (4H, t, J = 5.6 Hz).

(Reference Example 5-7)

trans-[1-(4-Chloro-7H-pyrrolo[2,3-d]pyrimidin-5-ylmethyl)-3-methylpiperidin-4-yl]carbamic acid tert-butyl ester methanesulfonate

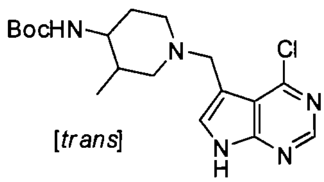
[*trans*]
MsOH

The compound of the Reference Example 5-7 was prepared according to the process for preparation described in the Reference Example 5-1 using the compound of the Reference Example 15-1.
Mass (EI, m/z): 380 $[M+1]^+$.
1H-NMR ($CD_3OD$) δ: 8.63 (1H, s), 7.91 (1H, s), 4.67 (2H, s), 3.72-3.53 (2H, m), 3.38-3.26 (1H, m), 3.20 (1H, td, J = 13.2, 2.4 Hz), 2.96 (1H, t, J = 12.6 Hz), 2.69 (3H, s), 2.18-2.04 (1H, m), 1.96-1.79 (1H, m), 1.70 (1H, qd, J = 13.2, 3.9 Hz), 1.43 (9H, s), 1.00 (3H, d, J = 6.3 Hz).

(Reference Example 5-8 [Enantiomer 1])

trans-[1-(4-Chloro-7H-pyrrolo[2,3-d]pyrimidin-5-ylmethyl)-3-methylpiperidin-4-yl]carbamic acid tert-butyl ester methanesulfonate [Enantiomer 1]

The compound of the Reference Example 5-8 [Enantiomer 1] was prepared according to the process for preparation described in the Reference Example 5-1 using the compound of the Reference Example 15-2 [Enantiomer 1]. The results of Mass and $^1$H-NMR were the same as those of the Reference Example 5-7.

(Reference Example 5-9 [Enantiomer 2])

trans-[1-(4-Chloro-7H-pyrrolo[2,3-d]pyrimidin-5-ylmethyl)-3-methylpiperidin-4-yl]carbamic acid tert-butyl ester methanesulfonate [Enantiomer 2]

The compound of the Reference Example 5-9 [Enantiomer 2] was prepared according to the process for preparation described in the Reference Example 5-1 using the compound of the Reference Example 15-3 [Enantiomer 2]. The results of Mass and $^1$H-NMR were the same as those of the Reference Example 5-7.

(Reference Example 6-1)

{1-[4-(3-Ethynylphenylamino)-7H-pyrrolo[2,3-d]pyrimidin-5-ylmethyl]-piperidin-4-yl}carbamic acid tert-butyl ester

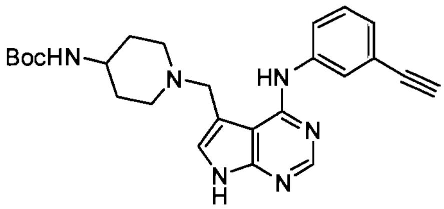

To a suspension of [1-(4-chloro-7H-pyrrolo[2,3-d]pyrimidin-5-ylmethyl)piperidin-4-yl]carbamic acid tert-butyl ester methanesulfonate (Reference Example 5-1) (1.50 g, 3.24 mmol) in 1,4-dioxane (6 ml) was added 3-ethynylaniline (1.5 ml, 13.3 mmol), and the mixture was stirred for 1 hour at 100 °C.

After the completion of the reaction, to the reaction mixture was added ethyl acetate (50 ml), and the organic layer was washed with saturated aqueous sodium hydrogen carbonate solution, dried over anhydrous sodium sulfate, and then concentrated under reduced pressure. The obtained residue was suspended in ethyl acetate, collected by filtration, and dried under reduced pressure to give the compound of the Reference Example 6-1 (653 mg, 1.46 mmol, yield 45%).

Mass (EI, m/z): 446 $[M]^+$.

$^1$H-NMR ($CDCl_3$) $\delta$: 11.00 (1H, s), 9.98 (1H, s), 8.44 (1H, s), 7.90 (1H, t, J = 1.7 Hz), 7.83 (1H, d, J = 8.3 Hz), 7.34 (1H, t, J = 7.9 Hz), 7.20 (1H, dt, J = 7.6, 1.2 Hz), 6.97 (1H, d, J = 2.0 Hz), 4.48 (1H, d, J = 7.8 Hz), 3.69 (2H, s), 3.59 (1H, br s), 3.11-3.03 (3H, m), 2.23 (2H, br s), 2.04 (2H, d, J = 10.7 Hz), 1.59-1.43 (11H, m).

Hereinafter, the compounds of the Reference Examples 6-2 to 6-18 were prepared according to the process for preparation described in the Reference Example 6-1.

Note that 3-bromo-5-fluoroaniline used in the preparation of the compound of the Reference Example 6-7 was prerared with reference to "Tehrani, Lida R. et al. Bioorganic & Medicinal Chemistry Letters, 15, 5061 (2005)". Also, in the preparation of the compounds of the Reference Examples 6-15 and 6-16, each preparation was performed after each corresponding pyrrolopyrimidine compound in the free form was converted into the salt form with an equivalent of methanesulfonic acid.

(Reference Example 6-2)

{1-[4-(3-Bromo-4-methylphenylamino)-7H-pyrrolo[2,3-d]pyrimidin-5-ylmethyl]piperidin-4-yl}carbamic acid tert-butyl ester

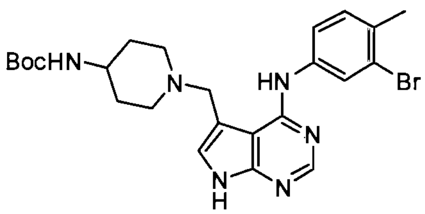

Mass (EI, m/z): 514, 516 $[M]^+$.

$^1$H-NMR ($CDCl_3$) $\delta$: 10.96 (1H, s), 9.14 (1H, br s), 8.41 (1H, s), 7.99 (1H, d, J = 2.2 Hz), 7.62 (1H, dd, J = 8.2, 2.2 Hz), 7.22 (1H, d, J = 8.2 Hz), 6.93 (1H, d, J = 2.2 Hz), 4.46 (1H, d, J = 7.6 Hz), 3.68 (2H, s), 3.59 (1H, br s), 3.05 (2H, d, J = 9.3 Hz), 2.39 (3H, s), 2.22 (2H, t, J = 12.3 Hz), 2.04 (2H, d, J = 10.3 Hz), 1.54-1.41 (11H, m).

(Reference Example 6-3)

{1-[4-(3,4,5-Tnfluorophenylammo)-7H-pyrrolo[2,3-d]pyrimidm-5-ylmethyl]piperidin-4-yl}carbamic acid tert-butyl ester

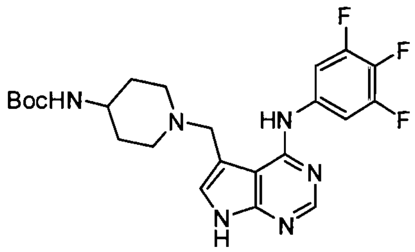

Mass (EI, m/z): 476 $[M]^+$.
$^1$H-NMR ($CDCl_3$) δ:11.06 (1H, s), 9.10 (1H, s), 8.44 (1H, s), 7.52 (2H, dd, J = 10.1, 6.2 Hz), 6.97 (1H, d, J = 2.2 Hz), 4.45 (1H, br s), 3.68 (2H, s), 3.55 (1H, br s), 3.05 (2H, br s), 2.22 (2H, br s), 2.05 (2H, d, J = 11.2 Hz), 1.49-1.45 (11H, m).

(Reference Example 6-4)

{1-[4-(3-Bromophenylamino)-7H-pyrrolo[2,3-d]pyrimidin-5-ylmethyl]piperidin-4-yl}carbamic acid tert-butyl ester

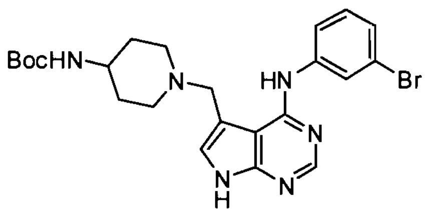

Mass (EI, m/z): 500, 502 $[M]^+$.
$^1$H-NMR ($CDCl_3$) δ: 11.02 (1H, s), 9.86 (1H, br s), 8.45 (1H, s), 8.06 (1H, t, J = 2.0 Hz), 7.69 (1H, d, J = 7.8 Hz), 7.24 (1H, t, J = 7.8 Hz), 7.18 (1H, dt, J = 8.0, 1.5 Hz), 6.97 (1H, d, J = 2.0 Hz), 4.47 (1H, d, J = 7.8 Hz), 3.69 (2H, s), 3.58 (1H, br s), 3.06 (2H, d, J = 12.2 Hz), 2.24 (2H, t, J = 11.8 Hz), 2.04 (2H, d, J = 10.7 Hz), 1.57-1.44 (11H, m).

(Reference Example 6-5)

{1-[4-(1H-Indazol-5-ylamino)-7H-pyrrolo[2,3-d]pyrimidin-5-ylmethyl)piperidin-4-yl]carbamic acid tert-butyl ester

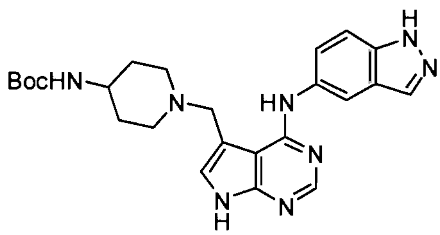

Mass (EI, m/z): 462 $[M]^+$.
$^1$H-NMR (DMSO-$d_6$) δ: 12.95 (1H, s), 11.53 (1H, d, J = 1.5 Hz), 10.93 (1H, s), 8.34 (1H, s), 8.22 (1H, s), 8.05 (1H, s), 7.53 (2H, s), 7.13 (1H, d, J = 2.0 Hz), 6.98 (1H, br d, J = 7.6 Hz), 3.66 (2H, s), 3.36-3.28 (1H, m), 3.02 (2H, br d, J = 11.7 Hz), 2.11 (2H, br t, J = 11.4 Hz), 1.80 (2H, br d, J = 10.7 Hz), 1.53 (2H, br dd, J = 21.1, 9.6 Hz), 1.38 (9H, s).

(Reference Example 6-6)

{1-[4-(3,4-Dichlorophenylamino)-7H-pyrrolo[2,3-d]pyrimidin-5-ylmethyl]piperidin-4-yl}carbamic acid tert-butyl ester

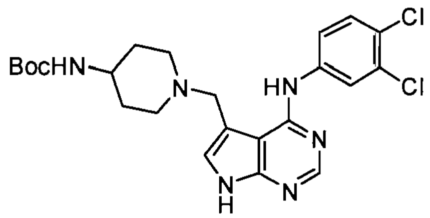

Mass (EI, m/z): 490 $[M]^+$.
$^1$H-NMR ($CDCl_3$) δ: 11.09 (1H, br s), 9.40 (1H, br s), 8.44 (1H, s), 8.06 (1H, d, J = 2.4 Hz), 7.60 (1H, dd, J = 8.5, 2.4 Hz), 7.41 (1H, d, J = 8.8 Hz), 6.97 (1H, d, J = 2.2 Hz), 4.46 (1H, br d, J = 8.5 Hz), 3.69 (2H, s), 3.65-3.50 (1H, m), 3.12-2.99 (2H, m), 2.30-2.17 (2H, m), 2.09-1.99 (2H, m), 1.51-1.43 (11H, m).

(Reference Example 6-7)

{1-[4-(3-Bromo-5-fluorophenylamino)-7H-pyrrolo[2,3-d]pyrimidin-5-ylmethyl]piperidin-4-yl}carbamic acid tert-butyl ester

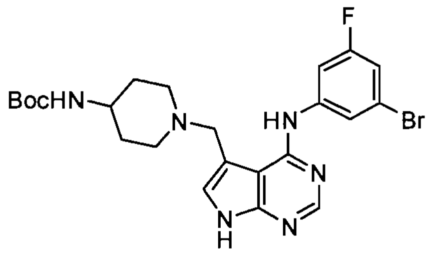

Mass (EI, m/z): 518, 520 $[M]^+$.
$^1$H-NMR ($CDCl_3$) δ: 11.15 (1H, br s), 9.17 (1H, br s), 8.47 (1H, s), 7.82 (1H, dt, J = 11.2, 2.1 Hz), 7.65-7.62 (1H, m), 6.99-6.97 (1H, m), 6.92 (1H, dt, J = 8.1, 2.0 Hz), 4.51-4.44 (1H, m), 3.69 (2H, s), 3.65-3.52 (1H, m), 3.14-2.99 (2H, m), 2.30-2.16 (2H, m), 2.06 (2H, d, J = 12.0 Hz), 1.49-1.44 (11H, m).

(Reference Example 6-8)

{1-[4-(3-Bromophenylamino)-7H-pyrrolo[2,3-d]pyrimidin-5-ylmethyl]-4-methylpiperidin-4-yl}carbamic acid tert-butyl ester

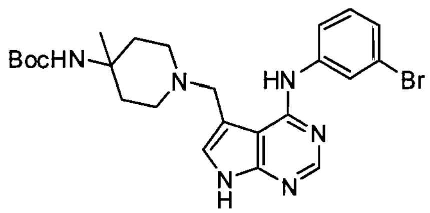

Mass (EI, m/z): 514, 516 $[M]^+$.
$^1$H-NMR ($CDCl_3$) δ: 11.14 (1H, s), 10.24 (1H, s), 8.44 (1H, s), 8.00 (1H, t, J = 1.8 Hz), 7.74 (1H, ddd, J = 7.9, 1.8, 1.4 Hz), 7.23 (1H, t, J = 7.9 Hz), 7.17 (1H, ddd, J = 7.9, 1.8, 1.4 Hz), 6.98 (1H, d, J = 1.7 Hz), 4.37 (1H, s), 3.71 (2H, s), 2.78

(2H, br s), 2.43 (2H, br s), 2.11 (2H, d, J = 12.9 Hz), 1.72 (2H, t, J = 11.5 Hz), 1.45 (9H, s), 1.39 (3H, s).

(Reference Example 6-9)

{1-[4-(3-Methoxyphenylamino)-7H-pyrrolo[2,3-d]pyrimidin-5-ylmethyl]-4-methylpiperidin-4-yl}carbamic acid tert-butyl ester

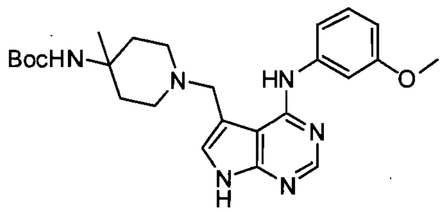

Mass (EI, m/z): 466 $[M]^+$.
$^1$H-NMR ($CDCl_3$) δ: 10.95 (1H, s), 9.94 (1H, s), 8.43 (1H, s), 7.52 (1H, d, J = 2.2 Hz), 7.31-7.22 (2H, m), 6.95 (1H, d, J = 2.0 Hz), 6.63 (1H, dt, J = 7.1, 2.1 Hz), 4.36 (1H, s), 3.85 (3H, s), 3.71 (2H, s), 2.78 (2H, br s), 2.44 (2H, br s), 2.09 (2H, d, J = 13.7 Hz), 1.74 (2H, t, J = 10.9 Hz), 1.45 (9H, s), 1.37 (3H, s).

(Reference Example 6-10)

{1-[4-(3-Ethynylphenylamino)-7H-pyrrolo[2,3-d]pyrimidin-5-ylmethyl.]-4-methylpiperidin-4-yl}carbamic acid tert-butyl ester

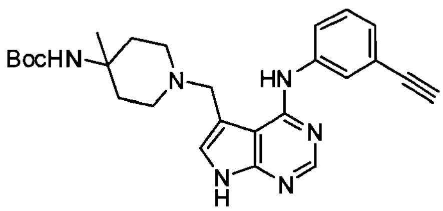

Mass (EI, m/z): 460 $[M]^+$.
$^1$H-NMR ($CDCl_3$) δ: 11.10 (1H, s), 10.44 (1H, s), 8.43 (1H, s), 7.87-7.83 (2H, m), 7.33 (1H, dd, J = 8.5, 7.6 Hz), 7.19 (1H, dt, J = 7.6, 1.2 Hz), 6.98 (1H, d, J = 2.0 Hz), 4.38 (1H, s), 3.71 (2H, s), 3.07 (1H, s), 2.78 (2H, br s), 2.44 (2H, br s), 2.11 (2H, d, J = 12.9 Hz), 1.73 (2H, t, J = 10.6 Hz), 1.45 (9H, s), 1.38 (3H, s).

(Reference Example 6-11)

{1-[4-(Benzofuran-5-ylamino)-7H-pyrrolo[2,3-d]pyrimidin-5-ylmethyl]-4-methylpiperidin-4-yl}carbamic acid tert-butyl ester

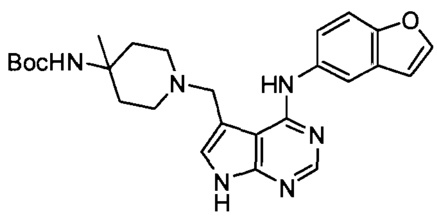

Mass (EI, m/z): 476 $[M]^+$.

$^1$H-NMR ($CDCl_3$) $\delta$: 11.03 (1H, s), 9.73 (1H, br s), 8.39 (1H, s), 8.08 (1H, d, J = 2.2 Hz), 7.62 (1H, d, J = 2.0 Hz), 7.50 (1H, d, J = 8.8 Hz), 7.44 (1H, dd, J = 8.8, 2.1 Hz), 6.94 (1H, d, J = 2.2 Hz), 6.78 (1H, dd, J = 2.1, 0.7 Hz), 4.36 (1H, s), 3.73 (2H, s), 2.78 (2H, br s), 2.46 (2H, br s), 2.12 (2H, d, J = 13.2 Hz), 1.70 (2H, t, J = 12.2 Hz), 1.45 (9H, s), 1.35 (3H, s).

(Reference Example 6-12)

{1-[4-(3-Bromophenylamino)-7H-pyrrolo[2,3-d]pyrimidin-5-ylmethyl]-4-methoxymethylpiperidin-4-yl}carbamic acid tert-butyl ester

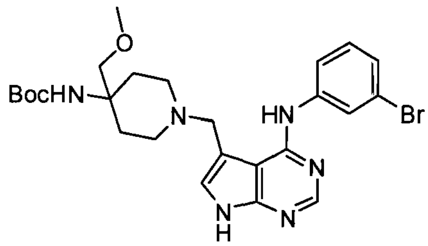

Mass (EI, m/z): 544, 546 $[M]^+$.
$^1$H-NMR ($CDCl_3$) $\delta$: 11.13 (1H, s), 9.06 (1H, br s), 8.43 (1H, s), 8.01 (1H, t, J = 1.6 Hz), 7.72 (1H, dt, J = 7.8, 1.6 Hz), 7.23 (1H, t, J = 7.8 Hz), 7.17 (1H, dt, J = 8.0, 1.6 Hz), 6.94 (1H, d, J = 2.0 Hz), 4.35 (1H, br s), 3.70 (2H, s), 3.51 (2H, s), 3.37 (3H, s), 2.85 (2H, br s), 2.39 (2H, br s), 2.15 (2H, d, J = 14.9 Hz), 1.87-1.78 (2H, m), 1.45 (9H, s).

(Reference Example 6-13)

{1-[4-(Benzofuran-5-ylamino)-7H-pyrrolo[2,3-d]pyrimidin-5-ylmethyl]-4-methoxymethylpiperidin-4-yl}carbamic acid tert-butyl ester

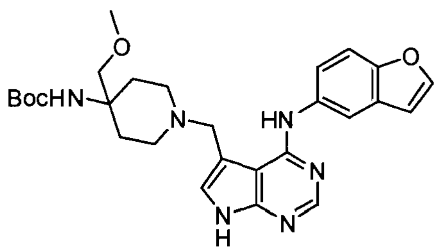

Mass (EI, m/z): 506 $[M]^+$.
$^1$H-NMR ($CDCl_3$) $\delta$: 11.05 (1H, s), 9.90 (1H, s), 8.39 (1H, s), 8.07 (1H, t, J = 1.2 Hz), 7.62 (1H, d, J = 2.1 Hz), 7.49 (2H, d, J = 1.2 Hz), 6.94 (1H, d, J = 2.1 Hz), 6.78 (1H, d, J = 2.2 Hz), 4.39 (1H, br s), 3.72 (2H, s), 3.49 (2H, s), 3.36 (3H, s), 2.87 (2H, d, J = 10.3 Hz), 2.41 (2H, t, J = 10.1 Hz), 2.14 (2H, d, J = 14.2 Hz), 1.90-1.80 (2H, m), 1.45 (9H, s).

(Reference Example 6-14)

{1-[4-(3-Bromophenylamino)-7H-pyrrolo[2,3-d]pyrimidin-5-ylmethyl]piperidin-4-yl}methylcarbamic acid tert-butyl ester

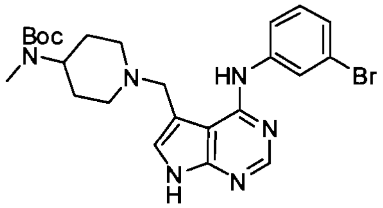

Mass (EI, m/z): 514, 516 $[M]^+$.
$^1$H-NMR ($CDCl_3$) δ:11.18 (1H, br s), 9.69 (1H, br s), 8.44 (1H, s), 7.94 (1H, t, J = 1.5 Hz), 7.88 (1H, d, J = 8.1 Hz), 7.24 (1H, t, J = 8.1 Hz), 7.18 (1H, dt, J = 8.1,1.5 Hz), 6.98 (1H, d, J = 1.7 Hz), 4.15 (1H, br s), 3.70 (2H, s), 3.19 (2H, d, J = 12.0 Hz), 2.70 (3H, s), 2.17 (2H, t, J = 10.5 Hz), 1.90-1.69 (4H, m), 1.46 (9H, s).

(Reference Example 6-15)

trans-4-Azide-1-[4-(3-bromophenylamino)-7H-pyrrolo[2,3-d]pyrimidin-5-ylmethyl]pipendin-3-ol

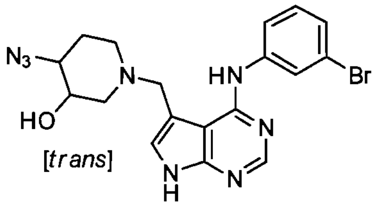

Mass (EI, m/z): 442, 444 $[M]^+$.
$^1$H-NMR ($CDCl_3$) δ: 10.89 (1H, br s), 10.55 (1H, s), 8.36 (1H, s), 8.03 (1H, t, J = 2.0 Hz), 7.51 (1H, dt, J = 6.8, 2.0 Hz), 7.22-7.19 (2H, m), 6.98 (1H, s), 3.79 (1H, d, J = 13.7 Hz), 3.70-3.61 (2H, m), 3.41-3.32 (1H, m), 3.16-3.05 (2H, m), 2.28 (1H, t, J = 11.1 Hz), 2.18-2.06 (2H, m), 1.73 (1H, dq, J = 4.0, 12.2 Hz).

(Reference Example 6-16)

[5-(1,4-Dioxa-8-azaspiro[4.5]dec-8-ylmethyl)-7H-pyrrolo[2,3-d]pyrimidin-4-yl](3-ethynylphenyl)amine

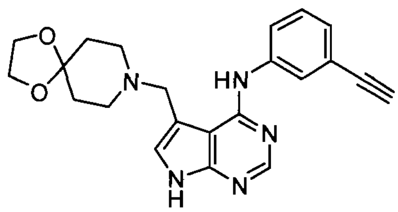

Mass (EI, m/z): 389 $[M]^+$.
$^1$H-NMR ($CDCl_3$) δ: 11.05 (1H, s), 10.26 (1H, br s), 8.44 (1H, s), 7.98 (1H, t, J = 1.7 Hz), 7.68 (1H, ddd, J = 8.1, 2.0, 1.1 Hz), 7.31 (1H, t, J = 7.8 Hz), 7.19 (1H, dt, J = 7.6, 1.2 Hz), 6.98 (1H, d, J = 2.0 Hz), 3.97 (4H, s), 3.73 (2H, s), 3.08 (1H, s), 2.75 (4H, br s), 1.84 (4H, t, J = 5.6 Hz).

(Reference Example 6-17)

{1-[4-(4-Methoxyphenylamino)-7H-pyrrolo[2,3-d]pyrimidin-5-ylmethyl]piperidin-4-yl]carbamic acid tert-butyl ester

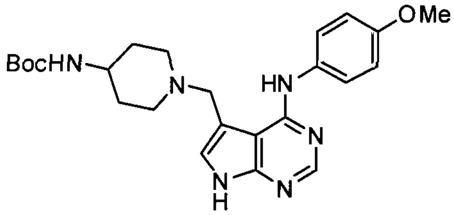

Mass (EI, m/z): 452 $[M]^+$.
1H-NMR ($CDCl_3$) δ: 10.80 (1H, br s), 10.10 (1H, s), 8.37 (1H, s), 7.62-7.55 (2H, m), 6.99-6.91 (3H, m), 4.45 (1H, d, J = 8.1 Hz), 3.83 (3H, s), 3.67 (2H, s), 3.56 (1H, br s), 3.14-2.94 (2H, m), 2.29-2.13 (2H, m), 2.01 (2H, d, J = 12.7 Hz), 1.57-1.35 (11H, m).

(Reference Example 6-18)

{1-[4-(4-Chlorophenylamino)-7H-pyrrolo[2,3-d]pyrimidin-5-ylmethyl]-4-methylpiperidin-4-yl]}carbamic acid tert-butyl ester

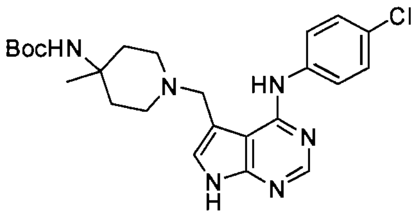

Mass (EI, m/z): 470 $[M]^+$.
1H-NMR ($CDCl_3$) δ: 11.04 (1H, s), 9.51 (1H, s), 8.41 (1H, s), 7.74-7.68 (2H, m), 7.36-7.30 (2H, m), 6.95 (1H, d, J = 2.0 Hz), 4.34 (1H, s), 3.71 (2H, s), 2.75 (2H, br s), 2.46 (2H, br s), 2.12 (2H, d, J = 12.7 Hz), 1.68 (2H, d, J = 11.0 Hz), 1.45 (9H, s), 1.36 (3H, s).

(Reference Example 6-19)

trans-{1-[4-(4-Chlorophenylamino)-7H-pyrrolo[2,3-d]pyrimidin-5-ylmethyl]-3-methylpiperidin-4-yl}carbamic acid tert-butyl ester

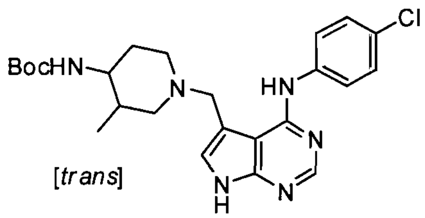

The compound of the Reference Example 6-19 was prepared according to the process for preparation described in the Reference Example 6-1 using the compound of the Reference Example 5-7.
Mass (EI, m/z): 470 $[M]^+$.
1H-NMR ($CDCl_3$) δ: 10.95 (1H, s), 9.10 (1H, s), 8.41 (1H, s), 7.72-7.65 (2H, m), 7.36-7.30 (2H, m), 6.94 (1H, d, J = 2.1 Hz), 4.32 (1H, d, J = 9.3 Hz), 3.71 (1H, d, J = 13.2 Hz), 3.63 (1H, d, J = 13.2 Hz), 3.31-3.17 (1H, m), 3.17-3.09 (1H, m), 3.09-3.00 (1H, m), 2.16 (1H, td, J = 12.3, 2.7 Hz), 2.06 (1H, ddt, J = 13.2, 3.9, 2.7 Hz), 1.84 (1H, t, J = 11.5 Hz), 1.63-1.39 (11H, m), 0.93 (3H, d, J = 6.3 Hz).

(Reference Example 6-20 [Enantiomer 1])

trans-{1-[4-(4-Chlorophenylamino)-7H-pyrrolo[2,3-d]pyrimidin-5-ylmethyl]-3-methylpiperidin-4-yl}carbamic acid tert-butyl ester [Enantiomer 1]

The compound of the Reference Example 6-20 [Enantiomer 1] was prepared according to the process for preparation described in the Reference Example 6-1 using the compound of the Reference Example 5-8 [Enantiomer 1]. The results of Mass and $^1$H-NMR were the same as those of the Reference Example 6-19.

(Reference Example 6-21 [Enantiomer 2])

trans-{1-[4-(4-Chlorophenylamino)-7H-pyrrolo[2,3-d]pyrimidin-5-ylmethyl]-3-methylpiperidin-4-yl}carbamic acid tert-butyl ester [Enantiomer 2]

The compound of the Reference Example 6-21 [Enantiomer 2] was prepared according to the process for preparation described in the Reference Example 6-1 using the compound of the Reference Example 5-9 [Enantiomer 2]. The results of Mass and $^1$H-NMR were the same as those of the Reference Example 6-19.

(Reference Example 7)

1-[4-(3-Ethynylphenylamino)-7H-pyrrolo[2,3-d]pyrimidin-5-ylmethyl]piperidin-4-one

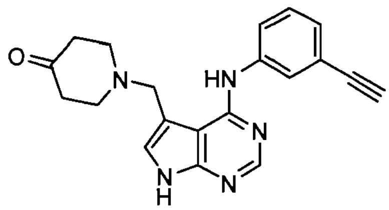

To a solution of [5-(1,4-dioxa-8-azaspiro[4.5]dec-8-ylmethyl)-7H-pyrrolo[2,3-d]pyrimidin-4-yl](3-ethynylphenyl) amine (Reference Example 6-16) (5.11 g, 13.1 mmol) in tetrahydrofuran (70 ml) was added 20 ml of 6 N aqueous hydrochloric acid solution (120 mmol), and the mixture was heated with being added acetone under reflux condition until all of the source material was consumed.

After the completion of the reaction, the reaction mixture was neutralized with 6 N aqueous sodium hydroxide solution, and to the mixture was added saturated aqueous sodium hydrogen carbonate solution, and extracted twice with ethyl acetate. The organic layer was dried over anhydrous sodium sulfate, and then concentrated under reduced pressure. The obtained residue was suspended in diisopropyl ether, and the solid was collected by filtration, washed with diisopropyl ether and hexane, and dried under reduced pressure to give the compound of the Reference Example 7 (4.37 g, 12.7 mmol, yield 96%).
Mass (EI, m/z): 345 $[M]^+$.
$^1$H-NMR ($CDCl_3$) δ: 10.77 (1H, s), 10.06 (1H, s), 8.47 (1H, s), 7.85 (1H, t, J = 1.7 Hz), 7.78 (1H, ddd, J = 8.1, 2.0, 1.1 Hz), 7.33 (1H, t, J = 7.8 Hz), 7.21 (1H, dt, J = 7.7, 1.3 Hz), 7.03 (1H, d, J = 2.0 Hz), 3.87 (2H, s), 3.08 (1H, s), 2.99 (4H, br s), 2.58 (4H, t, J = 6.1 Hz).

(Reference Example 8)

1-Benzoylpiperidine-4-carboxylic acid ethyl ester

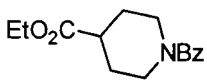

To a solution of piperidine-4-carboxylic acid ethyl ester (7.71 g, 50 mmol) in methylene chloride (200 ml) was

added triethylamine (21 ml, 150 mmol), and to the mixture was added a solution of benzoyl chloride (5.92 ml, 51 mmol) in methylene chloride (30 ml) dropwise with being stirred under ice-cold condition.

After the completion of the reaction, the reaction mixture was concentrated under reduced pressure, and ethyl acetate (50 ml) was added. The organic layer was washed sequentially with saturated aqueous ammonium chloride solution, distilled water, and saturated saline, dried over anhydrous magnesium sulfate, and then concentrated under reduced pressure. The obtained residue was suspended in a mixed solvent of ethyl acetate and diisopropyl ether, collected by filtration, and dried under reduced pressure to give the compound of the Reference Example 8 (11.3 g, 43 mmol, yield 86%).
Mass (CI, m/z): 262 $[M+1]^+$.
$^1H$-NMR ($CDCl_3$) δ: 7.43-7.37 (5H, m), 4.54 (1H, br s), 4.16 (2H, q, J = 7.2 Hz), 3.75 (1H, s), 3.05 (2H, t, J = 11.2 Hz), 2.57 (1H, tt, J = 10.7, 4.0 Hz), 2.04-1.73 (4H, m), 1.27 (3H, t, J = 7.2 Hz).

(Reference Example 9-1)

1-Benzoyl-4-methylpiperidine-4-carboxylic acid ethyl ester

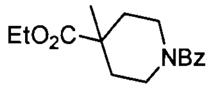

A solution of diisopropylamine (1.83 ml, 13 mmol) in tetrahydrofuran (30 ml) was cooled to -78 °C, and 1.6 M butyllithium/hexane solution (7.6 ml, 12 mmol) was added dropwise. The reaction solution was warmed to 0 °C, stirred for 10 minutes, and then cooled to -78 °C again, and a solution of 1-benzoylpiperidine-4-carboxylic acid ethyl ester (Reference Example 8) (2.61 g, 10 mmol) in tetrahydrofuran (10 ml) was added dropwise. The reaction solution was stirred for 1 hour at the same temperature, and then methyl iodide (0.81 ml, 13 mmol) was added dropwise, and warmed to room temperature.

After the completion of the reaction, the reaction mixture was concentrated under reduced pressure, and ethyl acetate (50 ml) was added. The organic layer was washed with saturated aqueous ammonium chloride solution, dried over anhydrous magnesium sulfate, and then concentrated under reduced pressure. The obtained oil was subjected to silica gel column chromatography (elution solvent: hexane:ethyl acetate = 4:1 to 2:1 (V/V)), and the fraction comprising the target compound was concentrated under reduced pressure to give the compound of the Reference Example 9-1 (1.86 g, 6.8 mmol, yield 68%).
Mass (EI, m/z): 275 $[M]^+$.
$^1H$-NMR ($CDCl_3$) δ: 7.42-7.35 (5H, m), 4.32 (1H, br s), 4.19 (2H, q, J = 7.1 Hz), 3.54 (1H, s), 3.13 (2H, br s), 2.19-2.08 (2H, br m), 1.57-1.29 (2H, m), 1.27 (3H, t, J = 7.2 Hz), 1.24 (3H, s).
Hereinafter, the compound of the Reference Example 9-2 was prepared according to the process for preparation described in the Reference Example 9-1.

(Reference Example 9-2)

1-Benzoyl-4-methoxymethylpiperidine-4-carboxylic acid ethyl ester

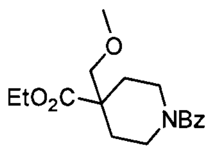

Mass (EI, m/z): 305 $[M]^+$.
$^1H$-NMR ($CDCl_3$) δ: 7.42-7.34 (5H, m), 4.44 (1H, br s), 4.22 (2H, q, J = 7.1 Hz), 3.62 (1H, br s), 3.41 (2H, br d, J = 12.0 Hz), 3.31 (3H, s), 3.23 (1H, br s), 3.03 (1H, br s), 2.15 (2H, br s), 1.59 (1H, br s), 1.44 (1H, br s), 1.28 (3H, t, J = 7.1 Hz).

(Reference Example 10-1)

1-Benzoyl-4-methylpiperidine-4-carboxylic acid

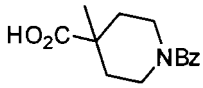

To a solution of 1-benzoyl-4-methylpiperidine-4-carboxylic acid ethyl ester (Reference Example 9-1) (1.86 g, 6.8 mmol) in ethanol (20 ml) was added 6 N aqueous sodium hydroxide solution (1.69 ml, 10.1 mmol), and the mixture was stirred for 5 hours at 50 °C.

After the completion of the reaction, the reaction mixture was concentrated under reduced pressure, ethyl acetate (30 ml) was added, and then the organic layer was extracted twice with distilled water (50 ml and 20 ml). To the combined aqueous layer was added concentrated hydrochloric acid until the pH became below 1, and extracted twice with ethyl acetate. The organic layer was dried over anhydrous magnesium sulfate, and then concentrated under reduced pressure. The obtained residue was suspended in a mixed solvent of ethyl acetate and diisopropyl ether, collected by filtration, and dried under reduced pressure to give the compound of the Reference Example 10-1 (1.26 g, 5.1 mmol, yield 75%).
Mass (CI, m/z): 248 $[M+1]^+$.
$^1$H-NMR ($CDCl_3$) $\delta$: 7.43-7.37 (5H, m), 4.34 (1H, br s), 3.57 (1H, br s), 3.21 (2H, br s), 2.23-2.05 (2H, m), 1.55-1.34 (2H, m), 1.29 (3H, s).
Hereinafter, the compound of the Reference Example 10-2 was prepared according to the process for preparation described in the Reference Example 10-1.

(Reference Example 10-2)

1-Benzoyl-4-methoxymethylpiperidine-4-carboxylic acid

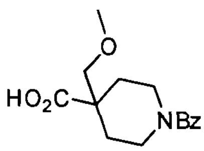

Mass (EI, m/z): 277 $[M]^+$.
$^1$H-NMR ($CDCl_3$) $\delta$: 7.43-7.37 (5H, m), 4.43 (1H, br s), 3.60 (1H, br s), 3.45 (2H, br d, J = 12.9 Hz), 3.35 (3H, s), 3.29 (1H, br s), 3.13 (1H, br s), 2.15 (2H, br s), 1.61 (1H, br s), 1.45 (1H, br s).

(Reference Example 11-1)

(1-Benzoyl-4-methylpiperidin-4-yl)carbamic acid tert-butyl ester

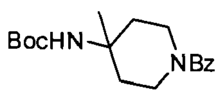

To a suspension of 1-benzoyl-4-methylpiperidine-4-carboxylic acid (Reference Example 10-1) (1.26 g, 5.1 mmol) in toluene (10 ml) were added triethylamine (2.13 ml, 15.2 mmol) and diphenylphosphorylazide (1.32 ml, 6.1 mmol), and the mixture was heated for 3 hours under reflux condition.

After the completion of the reaction, to the reaction mixture was added ethyl acetate (30 ml). The organic layer was washed sequentially with distilled water, saturated aqueous sodium carbonate solution, and saturated saline, dried over anhydrous magnesium sulfate, and then concentrated under reduced pressure.

To a solution of the obtained residue in tetrahydrofuran (10 ml) was added potassium tert-butoxide (1.12 g, 10.2 mmol), and the mixture was stirred for 20 minutes at room temperature.

After the completion of the reaction, to the reaction mixture was added ethyl acetate (30 ml). The organic layer was washed with saturated aqueous ammonium chloride solution, dried over anhydrous magnesium sulfate, and then concentrated under reduced pressure. The obtained residue was suspended in diisopropyl ether, collected by filtration, and dried under reduced pressure to give the compound of the Reference Example 11-1 (1.02 g, 3.2 mmol, yield 63%).
Mass (EI, m/z): 318 $[M]^+$.
$^1$H-NMR ($CDCl_3$) δ: 7.43-7.36 (5H, m), 4.38 (1H, br s), 4.16 (1H, br s), 3.45 (1H, br s), 3.33 (2H, br t, J = 11.5 Hz), 2.03 (2H, br s), 1.59 (2H, br s), 1.43 (9H, s), 1.38 (3H, s).
Hereinafter, the compound of the Reference Example 11-2 was prepared according to the process for preparation described in the Reference Example 11-1.

(Reference Example 11-2)

(1-Benzoyl-4-methoxymethylpiperidin-4-yl)carbamic acid tert-butyl ester

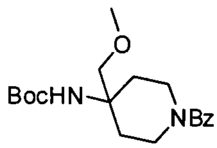

Mass (EI, m/z): 348 $[M]^+$.
$^1$H-NMR ($CDCl_3$) δ: 7.43-7.36 (5H, m), 4.42 (1H, s), 4.38 (1H, br s), 3.52 (1H, br s), 3.48 (2H, s), 3.36 (3H, s), 3.24 (2H, br s), 2.10 (2H, br d, J = 13.4 Hz), 1.70 (1H, br s), 1.55 (1H, br s), 1.43 (9H, s).

(Reference Example 12-1)

(4-Methylpiperidin-4-yl)carbamic acid tert-butyl ester

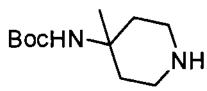

To a solution of (1-benzoyl-4-methylpiperidin-4-yl)carbamic acid tert-butyl ester (Reference Example 11-1) (318 mg, 1.0 mmol) in ethanol (3 ml) was added 6 N aqueous sodium hydroxide solution (1.0 ml, 6.0 mmol), and the mixture was heated for 8 hours under reflux condition.

After the completion of the reaction, the reaction mixture was concentrated under reduced pressure, and ethyl acetate (30 ml) was added. The organic layer was washed twice with distilled water, dried over anhydrous magnesium sulfate, and then concentrated under reduced pressure, and dried under reduced pressure to give the compound of the Reference Example 12-1 (145 mg, 0.67 mmol, yield 67%).
Mass (EI, m/z): 214 $[M]^+$.
$^1$H-NMR ($CDCl_3$) δ: 4.37 (1H, br s), 2.84-2.80 (4H, m), 1.91 (2H, dt, J = 13.3, 3.9 Hz), 1.51 (2H, td, J = 12.9, 6.2 Hz), 1.44 (9H, s), 1.34 (3H, s).
Hereinafter, the compound of the Reference Example 12-2 was prepared according to the process for preparation described in the Reference Example 12-1.

(Reference Example 12-2)

(4-Methoxymethylpiperidin-4-yl)carbamic acid tert-butyl ester

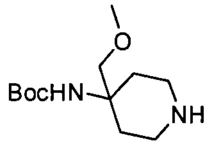

Mass (CI, m/z): 245 $[M+1]^+$.
$^1$H-NMR ($CDCl_3$) $\delta$: 4.40 (1H, br s), 3.49 (2H, s), 3.36 (3H, s), 2.88-2.84 (4H, m), 2.03-1.93 (4H, m), 1.44 (9H, s).

(Reference Example 13)

trans-1-Benzyl-3-methylpiperidin-4-ylcarbamic acid tert-butyl ester (Reference Example 13-1), cis-1-benzyl-3-methylpiperidin-4-ylcarbamic acid tert-butyl ester (Reference Example 13-2)

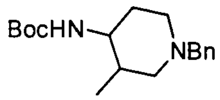

To 1-benzyl-3-methyl-4-piperidone (20.1 g, 98.7 mmol) were added sequentially 2 M ammonia in ethanol solution (250 ml, 500 mmol) and tetraisopropoxytitanium (57.8 ml, 197 mmol) under nitrogen atmosphere, and the mixture was stirred for 16 hours at room temperature. Then, the reaction solution was cooled to 0 °C, and to the solution was added sodium borohydride (5.77 g, 152 mmol) in several small portions. After the completion of the addition, the mixture was stirred for 3 hours at room temperature.

After the completion of the reaction, to the reaction solution was added water (250 ml) slowly at room temperature, and stirred for 30 minutes under the same condition. Subsequently, to the reaction solution was added an excess of Celite, and then the solid was filtered out using a Celite filter, and the filtered solid was washed twice with ethyl acetate (700 ml). After the filtrate and the wash solution were combined, the combined solution was separated into an organic phase and an aqueous phase, and the aqueous phase was extracted twice with ethyl acetate (300 ml). All of the organic phases were combined, and extracted twice with 1 N hydrochloric acid (250 ml). To the extract was added aqueous potassium carbonate solution until the pH became 9, and then the solution was extracted three times with ethyl acetate (600 ml). The obtained organic layer was dried over magnesium sulfate, and then concentrated under reduced pressure.

To a solution of the obtained residue (20.1 g) in dichloromethane (200 ml) were added sequentially triethylamine (16.5 ml, 118 mmol) and di-tert-butyl dicarbonate (26.1 ml, 114 mmol) under nitrogen atmosphere, and the mixture was stirred for 3.5 hours at room temperature.

After the completion of the reaction, to the mixture was added dichloromethane, washed one time with aqueous sodium hydrogen carbonate solution, and the obtained aqueous phase was extracted twice with ethyl acetate. The obtained organic layer was dried over magnesium sulfate, and then concentrated under reduced pressure. The obtained residue was subjected to silica gel column chromatography (elution solvent: dichloromethane-tetrahydrofuran), and the fraction comprising the target compound was concentrated under reduced pressure to give the each compound of the Reference Example 13-1 [trans isomer] (14.3 g, 46.8 mmol, yield 47%) and the Reference Example 13-2 [cis isomer] (12.0 g, 39.3 mmol, yield 40%).

(Reference Example 13-1 [trans isomer])

Rf value: 0.45 (chloroform:tetrahydrofuran = 4:1 (V/V)).
Mass (EI, m/z): 304 $[M]^+$.
$^1$H-NMR ($CDCl_3$) $\delta$: 7.37-7.19 (5H, m), 4.28 (1H, d, J = 8.5 Hz), 3.46 (2H, s), 3.22-3.05 (1H, m), 2.91-2.77 (2H, m), 2.01 (1H, td, J = 11.7, 2.4 Hz), 1.92 (1H, ddt, J = 12.6, 4.5, 2.4 Hz), 1.73 (1H, t, J = 11.1 Hz), 1.55-1.33 (11H, m), 0.89 (3H, d, J = 6.6 Hz).

(Reference Example 13-2 [cis isomer])

Rf value: 0.62 (chloroform:tetrahydrofuran = 4:1 (V/V)).
Mass (EI, m/z): 304 $[M]^+$.
$^1$H-NMR ($CDCl_3$) δ: 7.35-7.20 (5H, m), 4.56 (1H, d, J = 7.2 Hz), 3.72 (1H, br s), 3.49 (1H, d, J = 13.2 Hz), 3.42 (1H, d, J = 13.2 Hz), 2.50-2.27 (3H, m), 2.14 (1H, br s), 2.08-1.94 (1H, m), 1.79-1.65 (2H, m), 1.44 (9H, s), 0.92 (3H, d, J = 6.8 Hz).

(Reference Example 14)

trans-1-Benzyl-3-methylpiperidin-4-ylcarbamic acid tert-butyl ester [Enantiomer 1] (Reference Example 14-1 [Enantiomer 1]), trans-1-benzyl-3-methylpiperidin-4-ylcarbamic acid tert-butyl ester [Enantiomer 2] (Reference Example 14-2 [Enantiomer 2])

trans-1-Benzyl-3-methylpiperidin-4-ylcarbamic acid tert-butyl ester (Reference Example 13-1) (38.0 g, 124 mmol) was fractionated using a chiral column, and the optically active compounds of the Reference Example 14-1 [Enantiomer 1] (17.9 g, 58.8 mmol, yield 47%) and the Reference Example 14-2 [Enantiomer 2] (17.9 g, 58.8 mmol, yield 47%) were obtained.

(Reference Example 14-1 [Enantiomer 1])

The results of Mass and $^1$H-NMR were the same as those of the Reference Example 13-1. Optical purity: >99% ee
(Daicel CHIRALCEL OJ-H (trade name), Hexane/EtOH/$Et_2NH$ = 97/3/0.1 [v/v/v], 1.0 ml/min., 40 °C, 258 nm, retention time: 6.16 min.).

(Reference Example 14-2 [Enantiomer 2])

The results of Mass and $^1$H-NMR were the same as those of the Reference Example 13-1. Optical purity: >99% ee
(Daicel CHIRALCEL OJ-H, Hexane/EtOH/$Et_2NH$ = 97/3/0.1 [v/v/v], 1.0 ml/min., 40 °C, 258 nm, retention time: 5.15 min.).

(Reference Example 15)

trans-3-Methylpiperidin-4-ylcarbamic acid tert-butyl ester

(Reference Example 15-1)

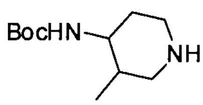

[*trans*]

To a solution of trans-1-benzyl-3-methylpiperidin-4-ylcarbamic acid tert-butyl ester (Reference Example 13-1) (8.24 g, 27.1 mmol) in methanol (100 ml) were added sequentially 20% palladium hydroxide/carbon [50% by weight of water content] (0.85 g) and ammonium formate (3.09 g, 49.0 mmol) under nitrogen atmosphere, and the mixture was heated for 70 minutes under reflux condition.
After the completion of the reaction, the reaction solution was cooled to room temperature, and the insoluble component was removed using a Celite filter, and then the filtrate was concentrated under reduced pressure to give the compound of the Reference Example 15-1 (crude product: 6.56 g). The obtained product was used in the next step without further purification.
Mass (EI, m/z): 214 $[M]^+$.
1H-NMR ($CDCl_3$) δ: 4.33 (1H, d, J = 6.6 Hz), 3.26-3.16 (1H, m), 3.06 (1H, dddd, J = 12.5, 4.4, 2.3, 1.5 Hz), 3.02 (1H, ddd, J = 11.6, 4.0, 1.5 Hz), 2.64 (1H, td, J = 12.5, 2.3 Hz), 2.32 (1H, t, J = 11.6 Hz), 1.96 (1H, ddt, J = 12.5, 4.4, 2.3 Hz), 1.38-1.28 (1H, m), 1.25 (1H, qd, J = 12.5, 4.4 Hz), 0.92 (3H, d, J = 6.6 Hz).

(Reference Example 15-2 [Enantiomer 1])

trans-3-Methylpiperidin-4-ylcarbamic acid tert-butyl ester [Enantiomer 1]

The compound of the Reference Example 15-2 [Enantiomer 1] was prepared according to the process for preparation described in the Reference Example 15-1 using the compound of the Reference Example 14-1 [Enantiomer 1]. The results of Mass and $^{1}$H-NMR were the same as those of the Reference Compound 15-1.

(Reference Example 15-3 [Enantiomer 2])

trans-3-Methylpiperidin-4-ylcarbamic acid tert-butyl ester [Enantiomer 2]

The compound of the Reference Example 15-3 [Enantiomer 2] was prepared according to the process for preparation described in the Reference Example 15-1 using the compound of the Reference Example 14-2 [Enantiomer 2]. The results of Mass and $^{1}$H-NMR were the same as those of the Reference Example 15-1.

[EXAMPLE]

(Example 1-1)

[5-(4-Aminopiperidin-l-ylmethyl)-7H-pyrrolo[2,3-d]pyrimidin-4-yl]benzofuran-5-ylamine

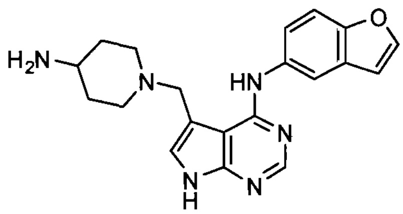

To a solution of {1-[4-(benzofuran-5-ylamino)-7H-pyrrolo[2,3-d]pyrimidin-5-ylmethyl]piperidin-4-yl}carbamic acid tert-butyl ester (Reference Example 3-1) (0.209 g, 0.452 mmol) in methylene chloride (3 ml) was added trifluoroacetic acid (1.5 ml, 20 mmol), and the mixture was stirred for 2 hours at room temperature.

After the completion of the reaction, the reaction mixture was concentrated under reduced pressure, and further subjected to azeotropy with toluene. The obtained residue was dissolved in methanol, and to the solution was added water, and then 2 N aqueous sodium hydroxide solution was added until the pH became 10 to 11. The precipitated solid was collected by filtration, and washed with water. The obtained solid was dissolved in methanol (2 ml) again, and to the solution was added water, and then stirred for 30 minutes at room temperature. The precipitated solid was collected by filtration, washed with water, and dried under reduced pressure to give the compound of the Example 1-1 (0.110 g, 0.303 mmol, yield 67%).
Mass (EI, m/z): 362 $[M]^{+}$.
$^{1}$H-NMR ($CD_3$ OD) $\delta$: 8.12 (1H, s), 7.96 (1H, d, J = 2.0 Hz), 7.75 (1H, d, J = 2.0 Hz), 7.51 (1H, d, J = 8.8 Hz), 7.44 (1H, dd, J = 8.8, 2.0 Hz), 7.04 (1H, s), 6.86 (1H, d, J = 2.0 Hz), 3.71 (2H, s), 3.08 (2H, br d, J = 11.7 Hz), 2.73 (1H, tt, J = 10.7, 3.9 Hz), 2.18 (2H, br t, J = 11.7 Hz), 1.88 (2H, br d, J = 12.0 Hz), 1.48 (2H, qd, J = 12.0, 3.0 Hz).
Hereinafter, the compounds of the Examples 1-2 to 1-20 were prepared according to the process for preparation described in the Example 1-1.

(Example 1-2)

[5-(4-Aminopiperidin-1-ylmethyl)-7H-pyrrolo[2,3-d]pyrimidin-4-yl](3-chloro-4-fluorophenyl)amine

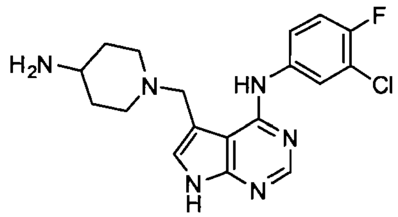

Mass (EI, m/z): 374 $[M]^+$.
$^1$H-NMR ($CD_3$ OD) δ: 8.23 (1H, s), 8.10 (1H, dd, J = 6.8, 2.7 Hz), 7.50 (1H, ddd, J = 9.0, 4.2, 2.7 Hz), 7.23 (1H, t, J = 9.0 Hz), 7.07 (1H, s), 3.70 (2H, d, J = 1.0 Hz), 3.07 (2H, br d, J = 12.2 Hz), 2.74 (1H, tt, J = 10.6, 4.0 Hz), 2.18 (2H, br t, J = 12.2 Hz), 1.89 (2H, br d, J = 12.3 Hz), 1.45 (2H, qd, J = 12.3, 4.0 Hz).

(Example 1-3)

[5-(4-Aminopiperidin-1-ylmethyl)-7H-pyrrolo[2,3-d]pyrimidin-4-yl](3-vinylphenyl)amine

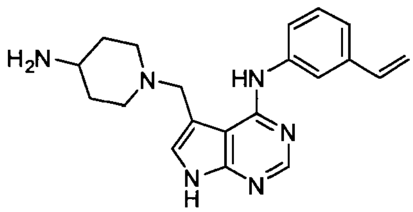

Mass (EI, m/z): 348 $[M]^+$.
$^1$H-NMR (DMSO-$d_6$) δ: 11.58 (1H, br s), 11.13 (1H, s), 8.26 (1H, s), 7.85-7.78 (2H, m), 7.76-7.73 (1H, m), 7.34 (1H, t, J = 7.8 Hz), 7.17-7.11 (2H, m), 6.74 (1H, dd, J = 17.8, 10.9 Hz), 5.83 (1H, dd, J = 17.6, 1.0 Hz), 5.29 (1H, dd, J = 10.9, 0.9 Hz), 3.66 (2H, s), 2.99 (2H, d, J = 11.5 Hz), 2.75-2.65 (1H, m), 2.11 (2H, t, J = 10.7 Hz), 1.78 (2H, d, J = 10.5 Hz), 1.44-1.29 (2H, m).

(Example 1-4)

[5-(4-Aminopiperidin-1-ylmethyl)-7H-pyrrolo[2,3-d]pyrimidin-4-yl](3-chlorophenyl)amine

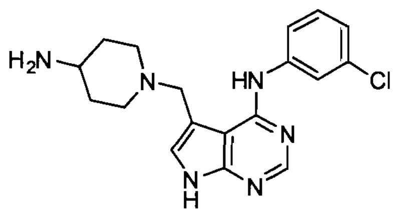

Mass (EI, m/z): 356 $[M]^+$.
$^1$H-NMR ($CD_3$ OD) δ: 8.24 (1H, s), 7.84 (1H, s), 7.49-7.47 (2H, m), 7.31-7.27 (2H, m), 3.92 (2H, s), 3.34-3.24 (3H, m), 2.41 (2H, br s), 2.10 (2H, d, J = 12.7 Hz), 1.67 (2H, dq, J = 4.0, 11.8 Hz).

(Example 1-5)

[5-(4-Aminopiperidin-1-ylmethyl)-7H-pyrrolo[2,3-d]pyrimidin-4-yl](3-ethynylphenyl)amine

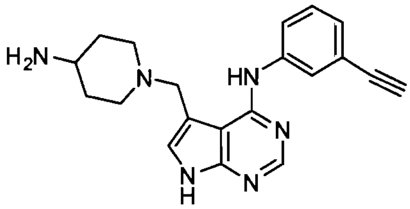

Mass (EI, m/z): 346 $[M]^+$.
$^1$H-NMR ($CD_3$ OD) δ: 8.22 (1H, s), 7.96 (1H, t, J = 1.9 Hz), 7.68 (1H, ddd, J = 8.1, 1.2, 1.9 Hz), 7.35 (1H, t, J = 8.1 Hz), 7.17 (1H, dt, J = 8.1, 1.2 Hz), 7.07 (1H, s), 3.71 (2H, d, J = 0.5 Hz), 3.49 (1H, s), 3.08 (2H, d, J = 12.0 Hz), 2.73 (1H, tt, J = 10.6, 4.2 Hz), 2.19 (2H, t, J = 10.7 Hz), 1.90 (2H, d, J = 10.3 Hz), 1.49 (2H, dq, J = 4.5, 11.9 Hz).

(Example 1-6)

[5-(4-Aminopiperidin-1-ylmethyl)-7H-pyrrolo[2,3-d]pyrimidin-4-yl](3-bromo-4-methylphenyl)amine

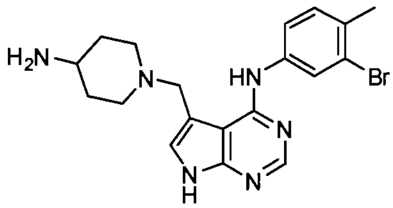

Mass (EI, m/z): 414, 416 $[M]^+$.
$^1$H-NMR ($CD_3$ OD) δ: 8.22 (1H, s), 8.12 (1H, d, J = 2.2 Hz), 7.50 (1H, dd, J = 8.3, 2.2 Hz), 7.27 (1H, d, J = 8.3 Hz), 7.06 (1H, s), 3.71 (2H, d, J = 0.5 Hz), 3.09 (2H, d, J = 12.7 Hz), 2.79 (1H, tt, J = 11.0, 4.0 Hz), 2.37 (3H, s), 2.19 (2H, t, J = 11.4 Hz), 1.92 (2H, d, J = 12.5 Hz), 1.50 (2H, dq, J = 4.0, 12.0 Hz).

(Example 1-7)

[5-(4-Aminopiperidin-1-ylmethyl)-7H-pyrrolo[2,3-d]pyrimidin-4-yl](3,4,5-trifluorophenyl)amine

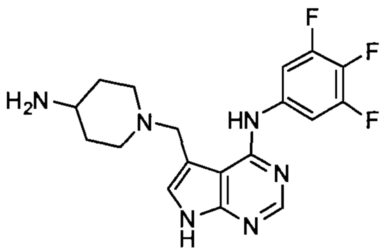

Mass (EI, m/z): 376 $[M]^+$.
$^1$H-NMR ($CD_3$ OD) δ: 8.29 (1H, s), 7.67-7.57 (2H, m), 7.10 (1H, s), 3.72 (2H, s), 3.08 (2H, d, J = 11.5 Hz), 2.75 (1H, tt, J = 10.5, 4.2 Hz), 2.19 (2H, t, J = 11.2 Hz), 1.90 (2H, d, J = 12.2 Hz), 1.44 (2H, dq, J = 4.0, 12.0 Hz).

(Example 1-8)

[5-(4-Aminopiperidin-1-ylmethyl)-7H-pyrrolo[2,3-d]pyrimidin-4-yl](3-bromophenyl)amine

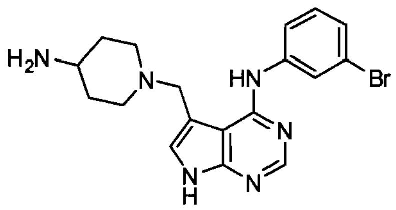

Mass (EI, m/z): 400, 402 $[M]^+$.
$^1$H-NMR ($CD_3OD$) δ: 8.25 (1H, s), 8.17 (1H, t, J = 2.0 Hz), 7.59 (1H, ddd, J = 8.0, 1.0, 2.0 Hz), 7.27 (1H, t, J = 7.9 Hz), 7.19 (1H, ddd, J = 7.9, 1.0, 2.0 Hz), 7.08 (1H, s), 3.71 (2H, s), 3.08 (2H, d, J = 12.2 Hz), 2.74 (1H, tt, J = 10.5, 4.2 Hz), 2.19 (2H, t, J = 11.2 Hz), 1.91 (2H, d, J = 11.5 Hz), 1.49 (2H, dq, J = 4.0, 12.0 Hz).

(Example 1-9)

[5-(4-Aminopiperidin-1-ylmethyl)-7H-pyrolo[2,3-d]pyrimidin-4-yl](1H-indazol-5-yl)amine

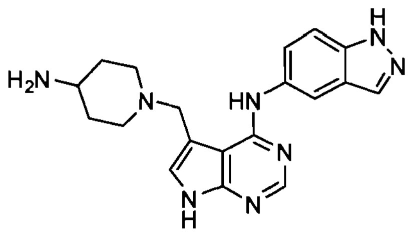

Mass (EI, m/z): 362 $[M]^+$.
$^1$H-NMR (DMSO-$d_6$) δ: 12.97 (1H, br s), 11.52 (1H, br s), 11.12 (1H, s), 8.35 (1H, s), 8.22 (1H, s), 8.04 (1H, s), 7.56-7.49 (2H, m), 7.12 (1H, s), 3.67 (2H, s), 2.98 (2H, br d, J = 11.2 Hz), 2.71-2.60 (1H, br m), 2.12 (2H, br t, J = 10.7 Hz), 1.77 (2H, br d, J = 10.3 Hz), 1.54 (2H, br s), 1.34 (2H, br q, J = 12.0 Hz).

(Example 1-10)

[5-(4-Aminopiperidin-1-ylmethyl)-7H-pyrrolo[2,3-d]pyrimidin-4-yl](3,4-dichlorophenyl)amine

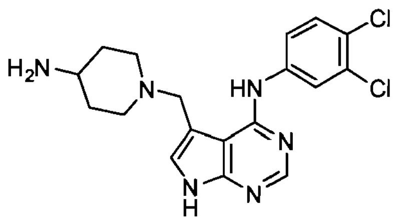

Mass (EI, m/z): 390 $[M]^+$.
$^1$H-NMR (DMSO-$d_6$) δ: 11.70 (1H, br s), 11.48 (1H, s), 8.34 (1H, d, J = 2.2 Hz), 8.33 (1H, s), 7.66 (1H, dd, J = 8.9, 2.3 Hz), 7.61 (1H, d, J = 8.8 Hz), 7.20 (1H, s), 3.67 (2H, s), 2.97 (2H, d, J = 10.5 Hz), 2.70-2.60 (1H, m), 2.11 (2H, t, J = 10.0 Hz), 1.76 (2H, d, J = 13.4 Hz), 1.35-1.20 (2H, m).

(Example 1-11)

[5-(4-Aminopiperidin-1-ylmethyl)-7H-pyrrolo[2,3-d]pyrimidin-4-yl](3-bromo-5-fluorophenyl)amine

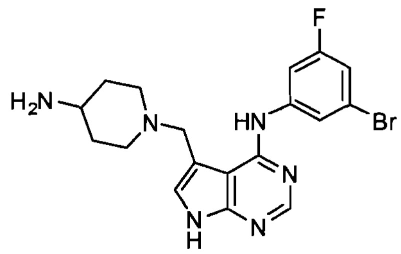

Mass (EI, m/z): 418, 420 $[M]^+$.
$^1$H-NMR ($CD_3$ OD) δ: 8.31 (1H, s), 7.80 (1H, dt, J = 11.2, 2.1 Hz), 7.76 (1H, td, J = 1.8, 0.8 Hz), 7.11 (1H, s), 6.97 (1H, ddd, J = 8.2, 2.3, 1.7 Hz), 3.73 (2H, s), 3.09 (2H, d, J = 11.2 Hz), 2.80-2.70 (1H, m), 2.21 (2H, t, J = 11.5 Hz), 1.93 (2H, d, J = 12.0 Hz), 1.55-1.40 (2H, m).

(Example 1-12)

[5-(4-Amino-4-methylpiperidin-1-ylmethyl)-7H-pyrrolo[2,3-d]pyrimidin-4-yl](3-bromophenyl)amine

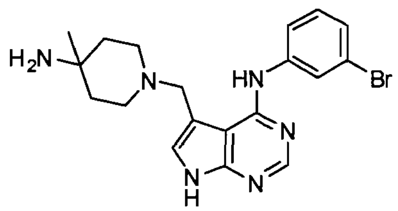

Mass (EI, m/z): 414,416 $[M]^+$.
$^1$H-NMR ($CD_3$ OD) δ: 8.24 (1H, s), 8.07 (1H, t, J = 2.0 Hz), 7.65 (1H, ddd, J = 7.9, 2.0, 1.0 Hz), 7.27 (1H, t, J = 8.1 Hz), 7.20 (1H, ddd, J = 8.0, 2.0, 1.0 Hz), 7.09 (1H, s), 3.76 (2H, d, J = 0.5 Hz), 2.65 (4H, br s), 1.70-1.64 (4H, m), 1.18 (3H, s).

(Example 1-13)

[5-(4-Amino-4-methylpiperidin-1-ylmethyl)-7H-pyrrolo[2,3-d]pyrimidin-4-yl](3-methoxyphenyl)amine

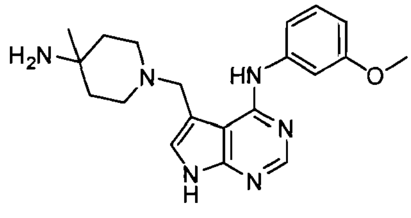

Mass (EI, m/z): 366 $[M]^+$.
$^1$H-NMR ($CD_3$ OD) δ: 8.18 (1H, s), 7.40 (1H, dd, J = 2.2, 2.0 Hz), 7.27 (1H, t, J = 8.1 Hz), 7.17 (1H, ddd, J = 8.1, 1.0, 2.0 Hz), 7.07 (1H, s), 6.67 (1H, ddd, J = 8.1, 1.0, 2.2 Hz), 3.82 (3H, s), 3.75 (2H, d, J = 0.7 Hz), 2.64 (4H, s), 1.71-1.62 (4H, m), 1.14 (3H, s).

(Example 1-14)

[5-(4-Amino-4-methylpiperidin-1-ylmethyl)-7H-pyrrolo[2,3-d]pyrimidin-4-yl](3-ethynylphenyl)amine

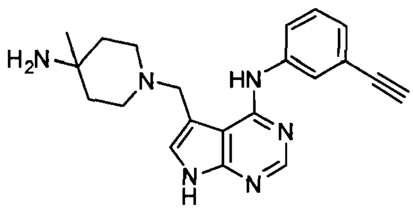

Mass (EI, m/z): 360 $[M]^+$.
$^1$H-NMR ($CD_3$ OD) δ: 8.23 (1H, s), 7.89 (1H, br s), 7.72 (1H, ddd, J = 8.0, 1.1, 2.0 Hz), 7.35 (1H, t, J = 8.0 Hz), 7.18 (1H, dt, J = 8.0, 1.1 Hz), 7.09 (1H, s), 3.77 (2H, s), 3.50 (1H, s), 2.68 (4H, s), 1.80-1.67 (4H, m), 1.23 (3H, s).

(Example 1-15)

[5-(4-Amino-4-methylpiperidin-1-ylmethyl)-7H-pyrrolo[2,3-d]pyrimidin-4-yl](benzofuran-5-yl)amine

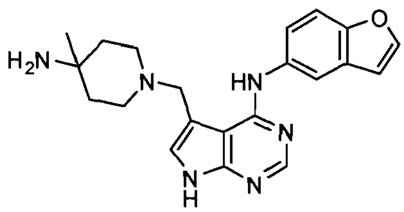

Mass (EI, m/z): 376 $[M]^+$.
$^1$H-NMR ($CD_3$ OD) δ: 8.12 (1H, s), 7.95 (1H, d, J = 2.0 Hz), 7.76 (1H, d, J = 2.2 Hz), 7.52 (1H, dt, J = 8.8, 0.7 Hz), 7.41 (1H, dd, J = 8.8, 2.0 Hz), 7.06 (1H, s), 6.85 (1H, dd, J = 2.3, 0.9 Hz), 3.76 (2H, d, J = 0.5 Hz), 2.65 (4H, s), 1.70-1.62 (4H, m), 1.15 (3H, s).

(Example 1-16)

[5-(4-Amino-4-methoxymethylpiperidin-1-ylmethyl)-7H-pyrrolo[2,3-d]pyrimidin-4-yl](3-bromophenyl)amine

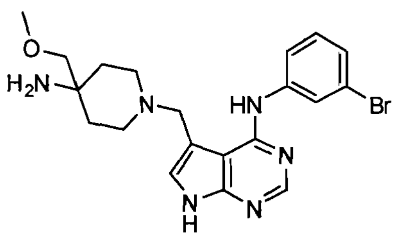

Mass (EI, m/z): 444, 446 $[M]^+$.
$^1$H-NMR ($CD_3$ OD) δ: 8.23 (1H, s), 8.04 (1H, t, J = 1.8 Hz), 7.65 (1H, ddd, J = 8.0, 1.8, 0.9 Hz), 7.26 (1H, t, J = 8.0 Hz), 7.20 (1H, ddd, J = 8.1, 1.8, 0.9 Hz), 7.08 (1H, s), 3.74 (2H, s), 3.36 (3H, s), 3.20 (2H, s), 2.76 (2H, br s), 2.54 (2H, br s), 1.82 (2H, ddd, J = 11.0, 13.9, 5.0 Hz), 1.54 (2H, d, J = 13.9 Hz).

(Example 1-17)

[5-(4-Amino-4-methoxymethylpiperidin-1-ylmethyl)-7H-pyrrolo[2,3-d]pyrimidin-4-yl](benzofuran-5-yl)amine

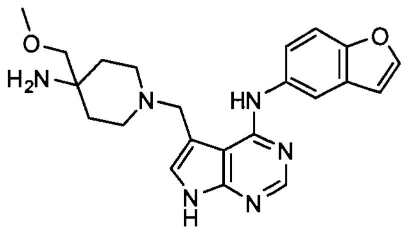

Mass (EI, m/z): 406 $[M]^+$.
$^1$H-NMR ($CD_3$ OD) δ: 8.11 (1H, s), 7.93 (1H, d, J = 2.0 Hz), 7.76 (1H, d, J = 2.2 Hz), 7.51 (1H, dt, J = 8.7, 0.8 Hz), 7.43 (1H, dd, J = 8.8, 2.2 Hz), 7.06 (1H, s), 6.84 (1H, dd, J = 2.2, 0.7 Hz), 3.76 (2H, s), 3.33 (3H, s), 3.18 (2H, s), 2.74 (2H, br s), 2.58 (2H, br s), 1.83 (2H, ddd, J = 13.8, 10.0, 5.0 Hz), 1.54 (2H, dt, J = 13.8, 4.0 Hz).

(Example 1-18)

(3-Bromophenyl)[5-(4-amino-4-methylaminopiperidin-1-ylmethyl)-7H-pyrrolo[2,3-d]pyrimidin-4-yl]amine

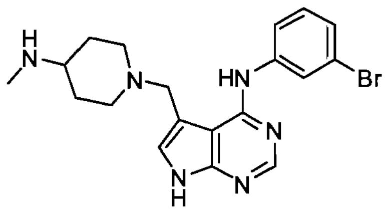

Mass (EI, m/z): 414,416 $[M]^+$.
$^1$H-NMR ($CD_3$ OD) δ: 8.25 (1H, s), 8.18 (1H, t, J = 2.0 Hz), 7.55 (1H, ddd, J = 8.0, 2.0, 1.0 Hz), 7.26 (1H, t, J = 8.0 Hz), 7.18 (1H, ddd, J = 8.0, 2.0, 1.0 Hz), 7.07 (1H, s), 3.70 (2H, s), 3.11 (2H, d, J = 12.2 Hz), 2.49 (1H, tt, J = 10.9,4.0 Hz), 2.36 (3H, s), 2.17 (2H, t, J = 11.2 Hz), 1.98 (2H, d, J = 12.7 Hz), 1.47 (2H, dq, J = 3.6, 12.1 Hz).

(Example 1-19)

[5-(4-Aminopiperidin-1-ylmethyl)-7H-pyrrolo[2,3-d]pyrimidin-4-yl](4-methoxyphenyl)amine

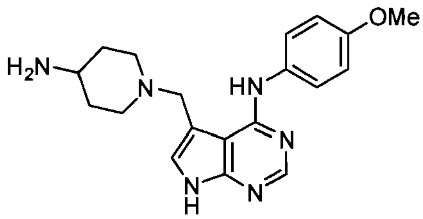

Mass (EI, m/z): 352 $[M]^+$.
$^1$H-NNM ($CD_3$ OD) δ: 8.08 (1H, s), 7.56-7.49 (2H, m), 7.02 (1H, s), 6.99-6.92 (2H, m), 3.81 (3H, s), 3.69 (2H, d, J = 0.6 Hz), 3.06 (2H, d, J = 11.1 Hz), 2.73 (1H, tt, J = 10.8, 4.2 Hz), 2.16 (2H, t, J = 11.1 Hz), 1.88 (2H, d, J = 12.9 Hz), 1.55-1.38 (2H, m).

(Example 1-20)

5-[(4-Amino-4-methylpiperidin-1-yl)methyl]-N-(4-methoxyphenyl)-7H-pyrrolo[2,3-d]pyrimidin-4-amine

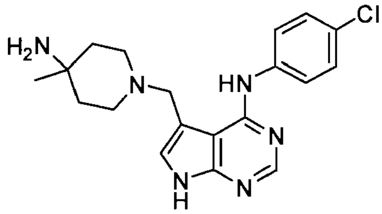

Mass (EI, m/z): 370 $[M]^+$.
$^1$H-NMR ($CD_3$ OD) δ: 8.19 (1H, s), 7.73-7.68 (2H, m), 7.38-7.33 (2H, m), 7.08 (1H, s), 3.75 (2H, d, J = 0.7 Hz), 2.65 (4H, br s), 1.68-1.58 (4H, m), 1.14 (3H, s).

(Example 1-21)

trans-[5-(4-Amino-3-methylpiperidin-1-ylmethyl)-7H-pyrrolo[2,3-d]pyrimidin-4-yl](4-chlorophenyl)amine

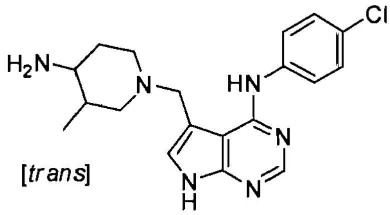

The compound of the Example 1-21 was prepared according to the process for preparation described in the Example 1-1 using the compound of the Reference Example 6-19. Mass (EI, m/z): 370 $[M]^+$.
$^1$H-NMR ($CD_3$ OD) δ: 8.19 (1H, s), 7.74-7.67 (2H, m), 7.3 8-7.31 (2H, m), 7.07 (1H, s), 3.69 (2H, s), 3.17-3.07 (1H, m), 3.01 (1H, ddd, J = 11.7, 3.9, 1.8 Hz), 2.3 3 (1H, ddd, J = 11.7, 9.6, 3.9 Hz), 2.15 (1H, td, J = 12.3, 2.4 Hz), 1.90 (1H, ddt, J = 13.2, 3.9, 2.4 Hz), 1.80 (1H, t, J = 11.7 Hz), 1.62-1.45 (2H, m), 0.95 (3H, d, J = 6.6 Hz).

(Example 1-22 [Enantiomer 1])

trans-[5-(4-Amino-3-methylpiperidin-1-ylmethyl)-7H-pyrrolo [2,3-d]pyrimidin-4-yl] (4-chlorophenyl)amine [Enantiomer 1]

The compound of the Example 1-22 [Enantiomer 1] was prepared according to the process for preparation described in the Example 1-1 using the compound of the Reference Example 6-20 [Enantiomer 1].
The results of Mass and $^1$H-NMR were the same as those of the Example 1-21.
Optical purity: >99% ee
(Daicel CHIRALCEL OD-H, Hexane/EtOH/$Et_2$NH = 80/20/0.1 [v/v/v], 1.0 ml/min., 25 °C, 270 nm, retention time: 8.11 min).

(Example 1-23 [Enantiomer 2])

trans-[5-(4-Amino-3-methylpiperidin-1-ylmethyl)-7H-pyrrolo[2,3-d]pyrimidin-4-yl](4-chlorophenyl)amine [Enantiomer 2]

The compound of the Example 1-23 [Enantiomer 2] was prepared according to the process for preparation described in the Example 1-1 using the compound of the Reference Example 6-21 [Enantiomer 2].
The results of Mass and $^1$H-NMR were the same as those of the Example 1-21.
Optical purity: >99% ee
(Daicel CHIRALCEL OD-H, Hexane/EtOH/$Et_2$NH = 80/20/0.1 [v/v/v], 1.0 ml/min., 25 °C, 270 nm, retention time: 9.72 min).

(Example 2-1)

trans-4-Amino-1-[4-(3-chloro-4-fluorophenylamino)-7H-pyrrolo[2,3-d]pyrimidin-5-ylmethyl]piperidin-3-ol

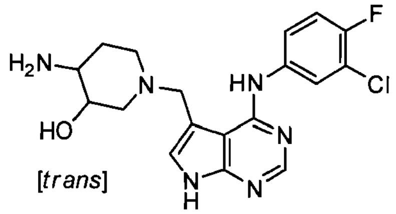

To a solution of 4-azide-1-[4-(3-chloro-4-fluorophenylamino)-7H-pyrrolo[2,3-d]pyrimidin-5-ylmethyl]piperidin-3-ol (Reference Example 3-6) (0.242 g, 0.580 mmol) in tetrahydrofuran (5 ml) were added triphenylphosphine (0.306 g, 1.17 mmol) and water (0.10 ml, 5.6 mmol) under nitrogen atmosphere at room temperature, and the mixture was stirred for 2.5 hours at 50 °C.

After the completion of the reaction, the reaction mixture was concentrated under reduced pressure, and the obtained residue was subjected to silica gel column chromatography (elution solvent: chloroform:methanol:28% aqueous ammonia solution = 20:1:0 to 6:1:0.07 (V/V/V)), and the fraction comprising the target compound was concentrated under reduced pressure. The obtained crude product was dissolved in methanol, and then to the solution was added water. The precipitated solid was collected by filtration, washed with water, and dried under reduced pressure to give the compound of the Example 2-1 (0.181 mg, 0.463 mmol, yield 80%).
Mass (EI, m/z): 390 $[M]^+$.
$^1$H-NMR ($CD_3$ OD) δ: 8.23 (1H, s), 8.13 (1H, dd, J = 6.6, 2.7 Hz), 7.43 (1H, ddd, J = 8.9, 4.0, 2.7 Hz), 7.22 (1H, t, J = 8.9 Hz), 7.10 (1H, s), 3.81 (1H, d, J = 13.4 Hz), 3.69 (1H, d, J = 13.4 Hz), 3.42-3.30 (1H, m), 3.19-3.02 (2H, m), 2.56 (1H, ddd, J = 11.4, 9.0, 3.9 Hz), 2.19 (1H, td, J = 12.0, 2.1 Hz), 2.02-1.86 (2H, m), 1.50 (1H, qd, J = 12.8, 3.9 Hz).
Hereinafter, the compounds of the Examples 2-2 to 2-3 were prepared according to the process for preparation described in the Example 2-1.

(Example 2-2)

trans-4-Amino-1-[4-(benzofuran-5-ylamino)-7H-pyrrolo[2,3-d]pyrimidin-5-ylmethyl]piperidin-3-ol

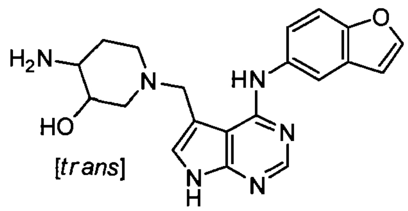

Mass (EI, m/z): 378 $[M]^+$.
$^1$H-NMR ($CD_3$ OD) δ: 8.12 (1H, s), 7.95 (1H, d, J = 1.7 Hz), 7.75 (1H, d, J = 2.1 Hz), 7.51 (1H, d, J = 8.7 Hz), 7.42 (1H, dd, J = 8.7, 2.1 Hz), 7.08 (1H, s), 6.86 (1H, d, J = 1.7 Hz), 3.81 (1H, d, J = 13.4 Hz), 3.72 (1H, d, J = 13.4 Hz), 3.41 (1H, td, J = 9.3, 4.2 Hz), 3.18 (1H, dd, J = 11.0, 4.2 Hz), 3.09 (1H, br d, J = 12.0 Hz), 2.56 (1H, ddd, J = 12.0, 9.3, 4.2 Hz), 2.18 (1H, td, J = 12.0, 2.3 Hz), 2.02-1.87 (2H, m), 1.54 (1H, qd, J = 12.0, 4.2 Hz).

(Example 2-3)

trans-4-Amino-1-[4-(3-bromophenylamino)-7H-pyrrolo[2,3-d]pyrimidin-5-ylmethyl]piperidin-3-ol

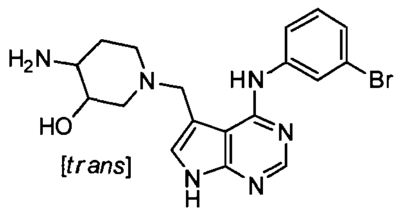

Mass (EI, m/z): 416,418 $[M]^+$.
$^1$H-NMR ($CD_3$ OD) δ: 8.25 (1H, s), 8.21 (1H, dd, J = 2.0, 2.0 Hz), 7.49 (1H, ddd, J = 8.0, 1.8, 1.0 Hz), 7.27 (1H, t, J = 7.9 Hz), 7.20 (1H, ddd, J = 8.0, 1.8, 1.0 Hz), 7.10 (1H, s), 3.81 (1H, d, J = 13.4 Hz), 3.69 (1H, d, J = 13.4 Hz), 3.38 (1H, td, J = 9.6, 4.2 Hz), 3.17-3.06 (2H, m), 2.55 (1H, ddd, J = 9.5, 11.6, 4.0 Hz), 2.19 (1H, ddd, J = 12.0, 2.5, 12.0 Hz), 1.98-1.91 (2H, m), 1.54 (1H, dq, J = 4.0, 12.5 Hz).

(Example 3-1)

(3-Ethynylphenyl)[5-(4-methylaminopiperidin-1-ylmethyl)-7H-pyrrolo[2,3-d]pyrimidin-4-yl]amine

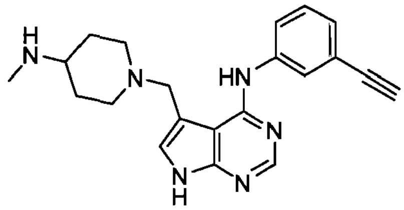

To a solution of 1-[4-(3-ethynylphenylamino)-7H-pyrrolo[2,3-d]pyrimidin-5-ylmethyl]piperidin-4-one (Reference Example 7) (0.75 g, 2.2 mmol) in tetrahydrofuran was added 40% aqueous methylamine solution (0.40 ml, 4.8 mmol), and the mixture was stirred for 30 minutes at room temperature. Then, to the mixture was added sodium triacetoxyhydroborate (0.75 g, 3.5 mmol), and stirred for 13 hours at room temperature.

After the completion of the reaction, to the reaction mixture was added methanol, and then concentrated under reduced pressure. The obtained residue was subjected to silica gel column chromatography (elution solvent: chloroform: methanol:28% aqueous ammonia solution = 9:1:0.01 to 4:1:0.01 (V/V/V)), and the fraction comprising the target compound was concentrated under reduced pressure to give the compound of the Example 3-1 (0.65 g, 1.8 mmol, yield 82%).
Mass (EI, m/z): 360 $[M]^+$.
$^1$H-NMR ($CD_3$ OD) δ: 8.23 (1H, s), 7.98 (1H, t, J = 1.7 Hz), 7.65 (1H, ddd, J = 8.1, 2.0, 1.1 Hz), 7.34 (1H, t, J = 7.9 Hz), 7.16 (1H, dt, J = 7.6, 1.2 Hz), 7.09-7.06 (1H, m), 3.72 (2H, s), 3.49 (1H, s), 3.13 (2H, d, J = 12.2 Hz), 2.56 (1H, tt, J = 11.0, 4.0 Hz), 2.39 (3H, s), 2.18 (2H, t, J = 11.7 Hz), 2.00 (2H, d, J = 12.2 Hz), 1.57-1.42 (2H, m).
Hereinafter, the compound of the Example 3-2 was prepared according to the process for preparation described in the Example 3-1.

(Example 3-2)

(3-Ethynylphenyl)[5-(4-prop-2-ynylaminopiperidin-1-ylmethyl)-7H-pyrrolo[2,3-d]pyrimidin-4-yl]amine

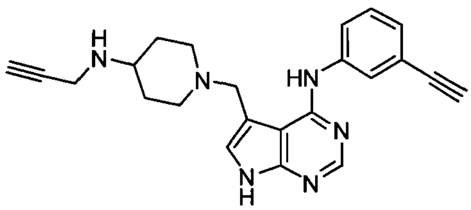

Mass (EI, m/z): 384 $[M]^+$.
$^1$H-NMR ($CDCl_3$) δ: 11.06 (1H, s), 9.89 (1H, s), 8.43 (1H, s), 7.96 (1H, t, J = 1.8 Hz), 7.76 (1H, ddd, J = 8.1, 2.0, 1.1 Hz), 7.31 (1H, t, J = 7.9 Hz), 7.18 (1H, dt, J = 7.7, 1.3 Hz), 6.96 (1H, s), 3.69 (2H, s), 3.47 (2H, d, J = 2.4 Hz), 3.07 (1H, s), 3.04 (2H, br s), 2.86 (1H, br m), 2.25 (2H, br t, J = 11.0 Hz), 2.21 (1H, t, J = 2.4 Hz), 1.95 (2H, br d, J = 10.5 Hz), 1.59-1.46 (2H, m).

(Formulation Example 1) Hard Capsule

An unit capsule is prepared by filling each of standard two-part hard gelatin capsules with 100 mg of the Example

Compound 1 in powder form, 150 mg of lactose, 50 mg of cellulose and 6 mg of magnesium stearate, washed, and then dried.

(Formulation Example 2) Soft Capsule

A soft capsule containing 100 mg of active ingredient is obtained by preparing a mixture of the Example Compound 1 in a digestive oil, for example, soybean oil, cottonseed oil or olive oil and injecting it into a gelatin using a positive displacement pump, washed, and then dried.

(Formulation Example 3) Tablet

A tablet is prepared according to a conventional method using 100 mg of the Example Compound 1, 0.2 mg of colloidal silicon dioxide, 5 mg of magnesium stearate, 275 mg of microcrystalline cellulose, 11 mg of starch and 98.8 mg of lactose. Also, if desired, a coating may be applied to the tablet.

(Test Example 1) HER2 kinase enzyme inhibitory activity (*in vitro*)

The HER2 kinase enzyme inhibitory activity of a compound of the present invention was evaluated by using a purified human recombinant HER2 kinase (Lys676-Val1255, GST fusion) and a synthetic peptide as a substrate, and detecting the phosphorylation level of the substrate by the HTRF method (homogeneous time resolved fluorescence method). Specifically, 25 ng/well of activated HER2, 200 μM of ATP, 200 ng/well of FLT3 analogous peptide (biotin label, peptide sequence is AGGSSDNEYFYVDF), and test compound (final concentration 0.01 nM to 30 μM) were mixed in kinase reaction buffer (60 mM HEPES, 5 mM $MgCl_2$, 5 mM $MnCl_2$, 3 mM $Na_3VO_4$, 1.25 mM DTT, pH 7.5) to prepare 50 μL/well of total reaction volume, and the mixture was reacted for 60 minutes at room temperature. Then, the phosphorylation reaction was stopped and HTRF reaction was carried out by adding 25 μL/well of PBS solution comprising the following components and reacting the mixture for 12 to 16 hours at 4 °C. The following components were dissolved in PBS (pH 7.0) to prepare the PBS solution composition: 60 mM EDTA (final concentration 20 mM), 1.2 M KF (final concentration 400 μM), 1 mg/mL BSA (final concentration 333 μg/mL), 6 μg/mL anti-PY20-Cryptate (Cisbio international, France, final concentration 2 μg/mL), and 15 μg/mL Streptavidin labeled with XL665 (Cisbio international, France, final concentration 5 μg/mL). The $IC_{50}$ value was calculated by using the average phosphorylation level of the FLT3 analogous peptide in a well containing no test compound (a group containing DMSO only) as 100%, calculating the T/C% (T/C = the phosphorylation level of a group containing a test compound / the phosphorylation level of a group containing DMSO only) of each group containing a test compound, and determining the concentration in which the T/C% became 50% as an approximate value from a linear function of two values which are above and below said concentration respectively.

The compounds with $IC_{50}$ value of less than 0.01 μM were the Example Compounds 1-6, 1-8, 1-10, 1-12, 1-13, 1-16, 1-18, 3-1, and 3-2, 0.01 μM or more to less than 0.05 μM were the Example Compounds 1-1 to 1-5, 1-7, 1-9, 1-11, 1-14, 1-15, and 2-1 to 2-3, 0.05 μM or more to less than 0.1 μM were the Example Compounds 1-17, 1-20, 1-21, and 1-22, and 0.1 μM or more were the Example Compounds 1-19 and 1-23.

(Test Example 2) Cytotoxic Activity (*in vitro*)

The cytotoxic activity of a compound of the present invention was investigated using a HER2 overexpressed gastric cancer cell line NCI-N87. Specifically, an appropriate amount of cells was seeded into a 96-well plate for cell culture at 50 μL/well, and precultured overnight at 37 °C under 5%-$CO_2$ condition. A solution of a test compound in DMSO with dilution series was diluted with medium, and added to the precultured cells at the volume of 50 μL/well. The mixture was prepared such that the final concentration of DMSO became 0.1 %. After the mixture was cultured for 72 hours at 37 °C under 5%-$CO_2$ condition, 100 μL/well of CellTiter-Glo solution (Promega, Madison, WI) was added, the luminescence was measured using a luminometer (Wallac ARVO™ SX) 10 minutes later, and the viable cell count was determined. The $GI_{50}$ value was calculated by using the average viable cell count in a well containing no test compound as 100%, calculating the T/C% (T/C = the viable cell count of a group containing a test compound / the viable cell count of a group containing no test compound) of each group containing a test compound, and determining the concentration in which the T/C% became 50% as an approximate value from a linear function of two values which are above and below said concentration respectively.

The compounds with $GI_{50}$ value less than 0.02 μM were the Example Compounds 1-3 to 1-5, 1-7, 1-8, 1-12 to 1-14, 1-16, 1-18, 2-3, 3-1, and 3-2, 0.02 μM or more to less than 0.05 μM were the Example Compounds 1-1, 1-2, 1-6, 1-10, 1-11, 1-15, 1-17, 1-20, 1-21, 1-22, 2-1, and 2-2, and 0.05 μM or more to less than 0.5 μM were the Example Compounds 1-9, 1-19, and 1-23.

(Test Example 3) *in vivo* Anti-tumor Effect

The anti-tumor effect *in vivo* of a compound of the present invention was evaluated using a HER2 overexpressed gastric cancer cell line. Specifically, a tumor fragment with about 5 mm square was transplanted subcutaneously using a transplantation needle, or an appropriate number of tumor cells was suspended in saline (Otsuka Pharmaceutical Co., Ltd.) and transplanted under the skin of the right axillary region of a nude mouse using a glass syringe equipped with a two-stage needle for subcutaneous injection. After the confirmation of the engraftment of the transplanted tumors, the mice were classified into groups depending on the tumor volume. The test compounds: the Example Compounds 1-1, 1-2, 1-5, 1-8, 1-13, 1-14, 1-15, 1-19, 1-20, 1-22, 2-1, 2-3, and 3-2 were each dissolved or suspended in an appropriate medium and orally administered forcedly to a mouse. The period of administration was varied from 2 weeks to 4 weeks depending on tumor, and the administration was carried out daily with drug holidays of Saturday and Sunday. The major axis (mm) and the minor axis (mm) of a tumor were measured sequentially using an electronic digital vernier caliper, and evaluated by the tumor growth inhibition ratio (GI%) on the judgement day (in principle, the next day of the final administration) using the following calculating formula. At the same time, the body weight was measured and the general status was observed sequentially, and the dosage that showed an effect and did not result in significant body weight loss or abnormality of appearance was defined as the effective dosage. As shown in the following table, all of the test compounds showed excellent anti-tumor effects.

[0357]

$$GI (\%) = (1 - A/B) \times 100$$

A: Average tumor volume on the judgement day of the group to which a compound was administered (*)
B: Average tumor volume on the judgement day of the untreated control group (*)
*: The tumor volume is calculated by 1/2 × [major axis of tumor] × [minor axis of tumor]$^2$.

(Table)
*in vivo* Anti-tumor Effect

[Table 1]

| Example No. | Dosage (mg/kg) | Tumor growth inhibition ratio (%) |
|---|---|---|
| 1-1 | 60 | 84 |
| 1-2 | 30 | 75 |
| 1-5 | 30 | 90 |
| 1-8 | 30 | 59 |
| 1-13 | 30 | 72 |
| 1-14 | 10 | 75 |
| 1-15 | 60 | 90 |
| 1-19 | 100 | 44 |
| 1-20 | 30 | 57 |
| 1-22 | 60 | 88 |
| 2-1 | 100 | 52 |
| 2-3 | 100 | 76 |
| 3-2 | 60 | 91 |

INDUSTRIAL APPLICABILITY

A compound of the present invention has a potent HER2 kinase inhibitory activity and an excellent anti-tumor activity, and thus is useful as a medicament, especially as an anti-tumor agent.

## Claims

1. A compound of the Formula (I):

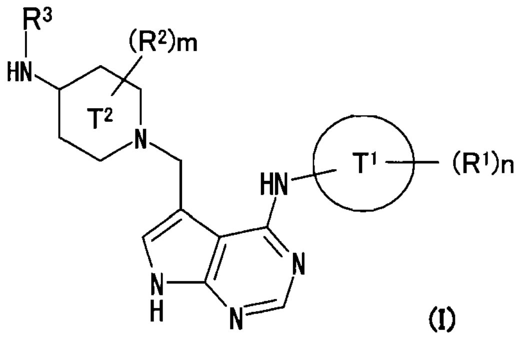

(I)

wherein,
$T^1$ is a phenol group, an indazolyl group, or a benzofuryl group,
$-(R^1)n$ represents that $T^1$ is substituted with the same or different n groups of $R^1$,
n is an integer of 0 to 3,
$R^1$ is a hydrogen atom, a halogen atom, a $C_{1-4}$ alkyl group, a $C_{2-6}$ alkenyl group, a $C_{2-6}$ alkynyl group, or a $C_{1-4}$ alkoxy group,
$T^2$ is a piperidinyl group,
$-(R^2)m$ represents that $T^2$ is substituted with the same or different m groups of $R^2$,
m is an integer of 0 to 3,
$R^2$ is a hydrogen atom, a hydroxy group, or a $C_{1-4}$ alkyl group, wherein said $C_{1-4}$ alkyl group may be substituted with one or two $C_{1-4}$ alkoxy groups,
$R^3$ is a hydrogen atom, a $C_{1-4}$ alkyl group, or a $C_{2-6}$ alkynyl group,
or a pharmacologically acceptable salt thereof.

2. The compound according to claim 1, wherein $R^1$ is a hydrogen atom, a fluorine atom, a chlorine atom, a bromine atom, a methyl group, an ethyl group, a vinyl group, an allyl group, an ethynyl group, a 1-propynyl group, a 2-propynyl group, a methoxy group, or an ethoxy group.

3. The compound according to claim 1 or claim 2, wherein $R^2$ is a hydrogen atom, a hydroxy group, a methyl group, an ethyl group, a methoxy group, an ethoxy group, a methoxymethyl group, an ethoxymethyl group, or a 2-methoxyethyl group.

4. The compound according to any one of claims 1 to 3, wherein $R^3$ is a hydrogen atom, a methyl group, an ethyl group, or a 2-propynyl group.

5. The compound according to any one of claims 1 to 3, wherein $R^3$ is a hydrogen atom.

6. The compound according to claim 1, wherein the group of the following Formula (II) in the Formula (I),

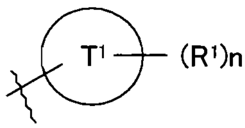

(II)

is the group of the following Formula (IIa)

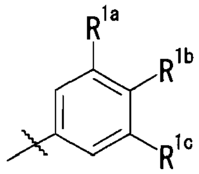

(IIa)

wherein $R^{1a}$, $R^{1b}$, and $R^{1c}$ are each the same or different and each is a hydrogen atom, a halogen atom, a $C_{1-4}$ alkyl group, a $C_{2-6}$ alkenyl group, a $C_{2-6}$ alkynyl group, or a $C_{1-4}$ alkoxy group,
or a benzofuranyl group.

**7.** The compound according to claim 6, wherein $R^{1a}$ is a halogen atom, a $C_{1-4}$ alkyl group, a $C_{2-6}$ alkenyl group, a $C_{2-6}$ alkynyl group, or a $C_{1-4}$ alkoxy group, and $R^{1b}$ and $R^{1c}$ are each a hydrogen atom.

**8.** The compound according to claim 6, wherein $R^{1a}$ and $R^{1c}$ are each a hydrogen atom, and $R^{1b}$ is a halogen atom, a $C_{1-4}$ alkyl group, a $C_{2-6}$ alkenyl group, a $C_{2-6}$ alkynyl group, or a $C_{1-4}$ alkoxy group.

**9.** The compound according to claim 6, wherein $R^{1a}$ and $R^{1b}$ are each the same or different and each is a halogen atom, a $C_{1-4}$ alkyl group, a $C_{2-6}$ alkenyl group, a $C_{2-6}$ alkynyl group, or a $C_{1-4}$ alkoxy group, and $R^{1c}$ is a hydrogen atom.

**10.** The compound according to claim 1, wherein the group of the following Formula (III) in the Formula (I),

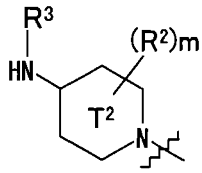

(III)

is the group of the following Formula (IIIa),

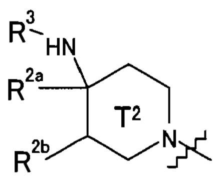

(IIIa)

wherein $R^{2a}$ and $R^{2b}$ are each the same or different and each is a hydrogen atom, a hydroxy group, or a $C_{1-4}$ alkyl group, wherein said $C_{1-4}$ alkyl group may be substituted with one or two $C_{1-4}$ alkoxy groups.

**11.** The compound according to claim 10, wherein $R^{2a}$ and $R^{2b}$ are each the same or different and each is a hydrogen atom or a methyl group.

**12.** The compound according to claim 10 or claim 11, wherein $R^3$ is a hydrogen atom.

**13.** The compound according to claim 10, wherein the group of the Formula (III) is any one group selected from the following (IIIb) group,

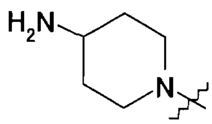

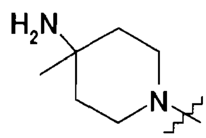

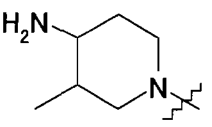 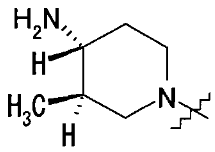 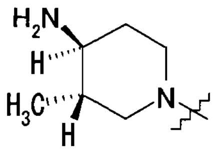

(IIIb)

.

**14.** The compound according to claim 1, wherein the compound of the Formula (I) is any one compound of the following (IV) group,

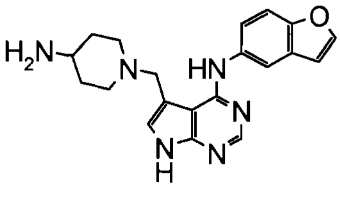 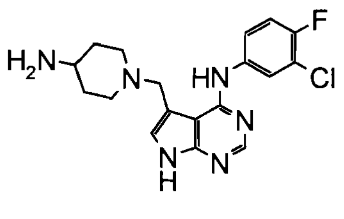 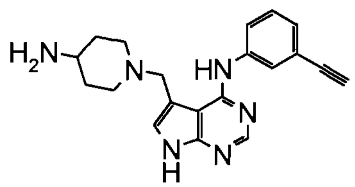

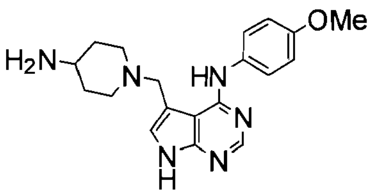 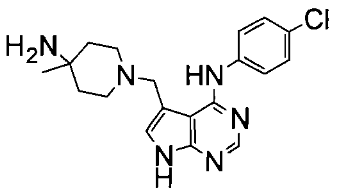 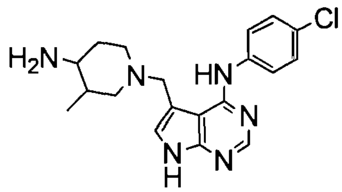

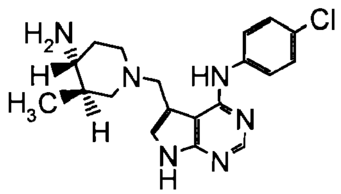 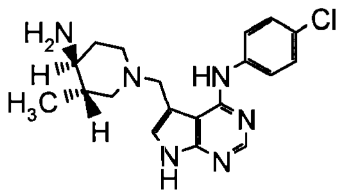

(IV)

.

**15.** A pharmacologically acceptable salt of the compound according to any one of claims 2 to 14.

**16.** A pharmaceutical composition comprising the compound according to any one of claims 1 to 14, or a pharmacologically acceptable salt thereof as an active ingredient.

**17.** A HER2 inhibitor comprising the compound according to any one of claims 1 to 14, or a pharmacologically acceptable salt thereof as an active ingredient.

**18.** An anti-tumor agent comprising the compound according to any one of claims 1 to 14, or a pharmacologically acceptable salt thereof as an active ingredient.

**19.** The anti-tumor agent according to claim 18, wherein the tumor is a blood cancer such as leukemia, lymphoma, or multiple myeloma, brain tumor, head and neck cancer, esophageal cancer, gastric cancer, appendix cancer, colon cancer, anal cancer, gallbladder cancer, bile duct cancer, pancreatic cancer, gastrointestinal stromal tumor, small cell lung cancer, non-small cell lung cancer, liver cancer, mesothelioma, thyroid cancer, kidney cancer, prostate cancer, neuroendocrine tumor, melanoma, breast cancer, endometrial cancer, cervical cancer, ovarian cancer, osteosarcoma, soft tissue sarcoma, Kaposi's sarcoma, myosarcoma, kidney cancer, bladder cancer, salivary gland cancer and/or testicular cancer.

## INTERNATIONAL SEARCH REPORT

International application No.
PCT/JP2010/062602

A. CLASSIFICATION OF SUBJECT MATTER
*C07D487/04*(2006.01)i, *A61K31/519*(2006.01)i, *A61P35/00*(2006.01)i, *A61P35/02*(2006.01)i, *A61P43/00*(2006.01)i

According to International Patent Classification (IPC) or to both national classification and IPC

B. FIELDS SEARCHED

Minimum documentation searched (classification system followed by classification symbols)
C07D487/04, A61K31/519, A61P35/00, A61P35/02, A61P43/00

Documentation searched other than minimum documentation to the extent that such documents are included in the fields searched

| | | | |
|---|---|---|---|
| Jitsuyo Shinan Koho | 1922-1996 | Jitsuyo Shinan Toroku Koho | 1996-2010 |
| Kokai Jitsuyo Shinan Koho | 1971-2010 | Toroku Jitsuyo Shinan Koho | 1994-2010 |

Electronic data base consulted during the international search (name of data base and, where practicable, search terms used)
CAplus/REGISTRY(STN)

C. DOCUMENTS CONSIDERED TO BE RELEVANT

| Category* | Citation of document, with indication, where appropriate, of the relevant passages | Relevant to claim No. |
|---|---|---|
| A | JP 2007-518721 A (BRISTOL-MYERS SQUIBB CO.), 12 July 2007 (12.07.2007), claims & WO 2005/066176 A1 & US 2005/182058 A1 & EP 1699797 A1 & US 2006/264438 A1 & US 7141571 B2 & US 7297695 B2 & US 2009/048244 A1 & EP 2058314 A1 & EP 1699797 B1 | 1-19 |
| A | JP 2005-509030 A (BRISTOL-MYERS SQUIBB CO.), 07 April 2005 (07.04.2005), claims & WO 2003/042172 A2 & US 2003/186983 A1 & EP 1446401 A2 & US 6908916 B2 & US 2005/209235 A1 & JP 4301440 B2 | 1-19 |

☒ Further documents are listed in the continuation of Box C.  ☐ See patent family annex.

* Special categories of cited documents:
"A" document defining the general state of the art which is not considered to be of particular relevance
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| 22 September, 2010 (22.09.10) | 05 October, 2010 (05.10.10) |
| Name and mailing address of the ISA/ Japanese Patent Office | Authorized officer |
| Facsimile No. | Telephone No. |

Form PCT/ISA/210 (second sheet) (July 2009)

**INTERNATIONAL SEARCH REPORT**

International application No. PCT/JP2010/062602

C (Continuation). DOCUMENTS CONSIDERED TO BE RELEVANT

| Category* | Citation of document, with indication, where appropriate, of the relevant passages | Relevant to claim No. |
|---|---|---|
| A | WO 2006/104971 A1 (BRISTOL-MYERS SQUIBB CO.), 05 October 2006 (05.10.2006), claims & US 2006/217369 A1 & EP 1863792 A1 & US 7358256 B2 & EP 1863792 B1 | 1-19 |
| A | JP 2005-501077 A (Novartis AG.), 13 January 2005 (13.01.2005), claims; paragraph [0039] & WO 2003/013541 A1 & EP 1416935 A1 & US 2004/242600 A1 & US 2004/248911 A1 & US 2007/161632 A1 & US 7244729 B2 & US 7323469 B2 & EP 1416935 B1 & US 7390805 B2 & EP 1416935 B8 & JP 4147184 B2 | 1-19 |
| A | PENG, T. et al., Pharmacophore analysis of epidermal growth factor receptor tyrosine kinase inhibitors, Huaxue Xuebao, 2003, Vol.61, No.3, p.430-434, Table 1 | 1-19 |
| A | TRAXLER, P.M. et al, 4-(Phenylamino) pyrrolopyrimidines: Potent and Selective, ATP Site Directed Inhibitors of the EGF-Receptor Protein Tyrosine Kinase, Journal of Medicinal Chemistry, 1996, Vol.39, No.12, p.2285-2292, Table 1 | 1-19 |
| A | JP 09-323995 A (Pharmacia & Upjohn S.p.A.), 16 December 1997 (16.12.1997), claims & EP 795556 A1 | 1-19 |

Form PCT/ISA/210 (continuation of second sheet) (July 2009)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- WO 199702266 A
- US 6140332 A
- WO 199807726 A
- US 6180636 B
- WO 09016132 A
- WO 05066176 A


### Non-patent literature cited in the description


- *Stem Cells,* 1998, vol. 16 (6), 413-428
- Annals Of Oncology. 2001, vol. 12, S81-587
- *Proc Natl Acad Sci U.S.A.,* 1987, vol. 84 (20), 7159-7163
- *Cancer Treatment Reviews,* 2009, vol. 35 (2), 121-136
- IYAKUHIN no KAIHATSU. Design of Molecules. HIROKAWA SHOTEN, 1990, 163-198